(19) Europäisches Patentamt
European Patent Office
Office européen des brevets

(11) EP 3 622 942 B1

# (12) EUROPÄISCHE PATENTSCHRIFT

(45) Veröffentlichungstag und Bekanntmachung des Hinweises auf die Patenterteilung:
**17.01.2024 Patentblatt 2024/03**

(21) Anmeldenummer: **18194789.6**

(22) Anmeldetag: **17.09.2018**

(51) Internationale Patentklassifikation (IPC):
***A61K 6/887*** *(2020.01)*

(52) Gemeinsame Patentklassifikation (CPC):
(C-Sets verfügbar)
**A61K 6/887** (Forts.)

(54) **POLYMERWERKSTOFFE MIT UNGESÄTTIGTEN ÜBERTRAGUNGSREAGENZIEN**

POLYMERIC MATERIALS WITH UNSATURATED TRANSMISSION REAGENTS

MATIÈRE POLYMÈRE CONTENANT DES RÉACTIFS DE TRANSFERT NON SATURÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.03.2020 Patentblatt 2020/12**

(73) Patentinhaber:
- **Ivoclar Vivadent AG**
  **9494 Schaan (LI)**
- **Technische Universität Wien**
  **1040 Wien (AT)**

(72) Erfinder:
- **Moszner, Norbert**
  **9495 Triesen (LI)**
- **Lamparth, Iris**
  **9472 Grabs (CH)**
- **Catel, Yohann**
  **9497 Buch (CH)**
- **Liska, Robert**
  **2123 Schleinbach (AT)**
- **Gorsche, Christian**
  **1140 Wien (AT)**
- **Peer, Gernot**
  **1030 Wien (AT)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 2 965 741** **WO-A1-88/04304**
**WO-A2-2012/112350**

- **MEIJS G F ET AL: "THE USE OF ACTIVATED BENZYL ETHERS TO CONTROL MOLECULAR WEIGHT IN FREE POLYMERIZATIONS", MAKROMOL. CHEM AND PHYSICS, WILEY, Bd. 191, 1. Januar 1990 (1990-01-01), Seiten 1545-1553, XP001062801, ISSN: 0025-116X, DOI: 10.1002/MACP.1990.021910707**
- **Parag K. Shah ET AL: "Application of an addition-fragmentation-chain transfer monomer in di(meth)acrylate network formation to reduce polymerization shrinkage stress", Polymer Chemistry, vol. 8, no. 30, 1 January 2017 (2017-01-01), pages 4339-4351, XP055583442, ISSN: 1759-9954, DOI: 10.1039/C7PY00702G**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

**(Forts. nächste Seite)**

(52) Gemeinsame Patentklassifikation (CPC): (Forts.)

C-Sets
**A61K 6/887, C08L 33/10**

**Beschreibung**

Die vorliegende Erfindung betrifft ungesättigte Übertragungsreagenzien und radikalisch polymerisierbare Zusammensetzungen, die sich als Werkstoffe für technische und medizinische Anwendungen eignen, beispielsweise in der Chirurgie oder Augenheilkunde und besonders als Dentalwerkstoffe, z.B. als Prothesenmaterialien, Zemente, Füllungskomposite, Verblendmaterialien oder als Werkstoffe für künstliche Zähne, Inlays, Onlays, Kronen und Brücken.

Radikalische Polymerisate werden durch radikalische Polymerisation von einem (Homopolymerisate) oder mehreren (Copolymerisate) radikalisch polymerisierbaren Monomeren gebildet. Dabei werden je nach der Funktionalität der polymerisierten Monomere lineare (bei monofunktionellen Monomeren) oder vernetzte (bei di- oder multifunktionellen Monomeren) Polymere erhalten. Die radikalische Polymerisation hat zum einen den Vorteil, dass sich viele technisch relevante Monomere, wie z.B. Ethylen, Styrol, Vinylchlorid, Acrylnitril, Vinylacetat, aber auch Diene, (Meth)acrylate und (Meth)acrylamide auf diese Weise polymerisieren lassen. Zum anderen lässt sich die radikalische Polymerisation in vielfältiger Weise durchführen, z.B. in Substanz (Bulkpolymerisation), Lösung, Suspension oder Emulsion. Zur Polymerisationsauslösung werden radikalbildende Initiatoren zugesetzt, die durch Thermolyse, Photolyse oder Redoxreaktion Radikale bilden.

Die radikalische Polymerisation läuft nach einem Kettenwachstumsmechanismus ab, bei dem sich die aus dem Initiator gebildeten polymerisationsauslösenden Primärradikale an die Doppelbindung der Monomere addieren. Die so gebildeten Startradikale addieren in einer schnellen Wachstumsreaktion viele weitere Monomermoleküle, bis das Wachstum der Polymerradikale durch Kombination oder Disproportionierung abgebrochen wird und so die fertigen Makromoleküle entstehen. Durch die Zugabe von Kettenüberträgern, sog. Übertragungsreagenzien oder Reglern, lässt sich die zahlenmittlere Molmasse des gebildeten Polymers gezielt regulieren (vgl. H. G. Elias, Makromoleküle, Bd. 1, 6. Aufl., Wiley-VCH, Weinheim etc. 199, 299-352).

Zu den bekannten Kettenüberträgern zählen beispielsweise Mercaptane, die durch Übertragung eines Wasserstoffatoms Thiyl-Radikale bilden, die dann wiederum die Bildung einer neuen Polymerisationskette initiieren. Als Kettenüberträger haben sich weiterhin doppelbindungshaltige Reagenzien besonders bewährt, die nach einem radikalischen Additions-Fragmentierungs-Kettenübertragungs-Mechanismus (engl.: "addition-fragmentation chain transfer", AFCT) reagieren. Als AFCT-Reagenzien sind Schwefelverbindungen, wie Allylsulfide, Allylsulfone, Vinylsulfonester, Dithioester, Dithiocarbamate, Xanthate und Trithiocarbonate besonders wirksam und gut untersucht (Moad et al., Polymer 49, 1079-1131 (2008)).

Die US 2,694,699 beschreibt die Homo- und Copolymerisation von $\alpha$-Sulfonyloxyacrylaten. Als Kettenregler können Alkylmercaptane zugesetzt werden.

Die US 5,932,675 offenbart ein Verfahren zur Herstellung von Polymeren mit geringem Molekulargewicht durch radikalische Polymerisation, wobei die Kontrolle des Molekulargewichts durch die Zugabe z.B. von $\alpha$-(t-Butylthiomethyl)styrol als Kettenübertragungsreagenz erfolgt.

Die EP 3 090 722 A1 beschreibt radikalisch polymerisierbare Dentalwerkstoffe, denen als Kettenübertragungsreagenz Sulfonsäureester zugesetzt werden.

Nachteilig an schwefelhaltigen Übertragungsreagenzien ist, dass sie zu Verfärbungen der Polymerisate führen können und/oder eine relativ hohe Zelltoxizität aufweisen.

Die US 9,320,685 B2 beschreibt schwefelfreie, ungesättigte Übertragungsreagenzien, wie z.B. die Verbindungen (2) oder (3), die nicht zu Verfärbungen führen und die Schrumpfungsspannung von dentalen Kompositen verringern sollen.

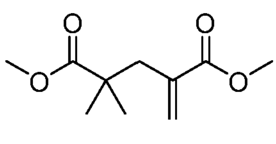

2

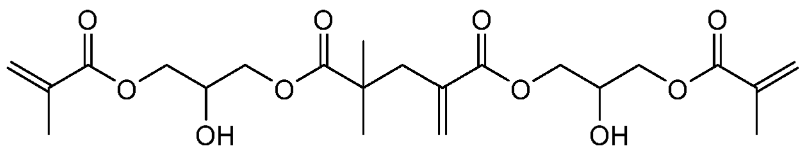

3

Jedoch weisen diese Übertragungsreagenzien den Nachteil eines geringeren Doppelbindungsumsatzes und einer verringerten Polymerisationsgeschwindigkeit auf (vgl. P. K. Shah, J. W. Stansbury, C. N. Bowman, Polym. Chem. 2017; DOI:10.1039/c7py0072g).

Die WO 88/04304 A1 offenbart ein Verfahren zur Herstellung von Polymeren durch freie radikalische Polymerisation von Zusammensetzungen, die eine Verbindung der Formel $H_2 = C < \genfrac{}{}{0pt}{}{Y}{R^1}$ enthalten. $R^1$ ist Wasserstoff oder eine Gruppe, die den Vinylkohlenstoff für die freie radikalische Addition aktiviert, und Y ist $-CH_2X(R^2)_n$ oder $-OR^2$. Diese Verbindungen sollen als Kettenübertragungsreagenz wirksam sein und eine konzentrationsabhängige Verringerung des

Molekulargewichts der hergestellten Polymeren bewirken.

EP 2 965 741 A1 offenbart radikalisch polymerisierbare Dentalmaterialien, die mindestens eine Verbindung der folgenden Formel als Kettenüberträger enthalten:

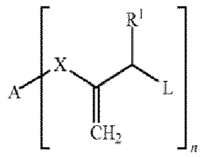

in der X -COO- oder -CON($R^{10}$)- ist oder entfällt. Vinylether werden nicht offenbart.

Meijs et al., Makromol Chem. 191, 1545-1553 (1990), beschreiben die Verwendung aktivierter Benzylvinylether als schwefelfreie Kettenüberträger zur Kontrolle des Molekulargewichts bei der radikalischen Polymerisation.

Die WO 2012/112350 A2 offenbart Dentalwerkstoffe, die ethylenisch ungesättigte Additions-Fragmentierungs-Reagenzien enthalten. Die Additions-Fragmentierungs-Reagenzien weisen eine ethylenisch ungesättigte Endgruppe und eine Hauptkette mit einer $\alpha,\beta$ -ungesättigten Carbonylgruppe auf.

Der Erfindung liegt die Aufgabe zugrunde, Dentalmaterialien bereitzustellen, die sich im Vergleich zu bekannten Materialien auf Basis multifunktioneller (Meth)acrylate nach der Härtung durch einen engeren Bereich des Glasüberganges, eine verbesserte Schlagzähigkeit und eine verringerte Polymerisationsschrumpfungskraft bei ähnlichen mechanischen Eigenschaften auszeichnen. Die Materialien sollen vor allem eine hohe Reaktivität und Aushärtungsgeschwindigkeit, keine Eigenfarbe und keinen störenden Geruch aufweisen.

Diese Aufgabe wird erfindungsgemäß durch radikalisch polymerisierbare Zusammensetzungen zur Anwendung bei der intraoralen Restauration geschädigter Zähne gemäß Anspruch 1 und die Verwendung radikalisch polymerisierbarer Zusammensetzungen zur extraoralen Herstellung oder Reparatur von Dentalrestaurationen oder als medizinischer Werkstoff zur Herstellung von Medizinprodukten für die Chirurgie oder für die Augenheilkunde gemäß Anspruch 2 gelöst.

Anspruch 11 bezieht sich auf die Verwendung von Verbindungen zur Verringerung der Polymerisationsschrumpfungsspannung und Anspruch 12 auf die Verwendung von Verbindungen zur Verbesserung der Schlagzähigkeit von radikalischen Polymerisaten.

Die radikalisch polymerisierbaren Zusammensetzungen enthalten mindestens einen Vinylether gemäß der allgemeinen Formel II oder III

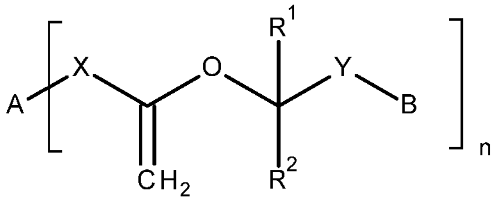

Formel II

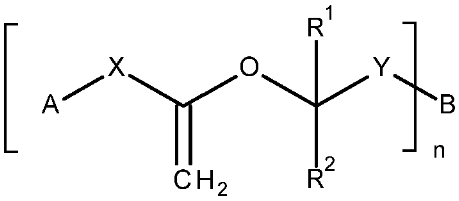

Formel III

und mindestens ein (Meth) acrylat mit zwei oder mehr radikalisch polymerisierbaren Gruppen oder eine Mischung von (Meth)acrylaten mit einer und (Meth)acrylaten mit zwei oder mehr radikalisch polymerisierbaren Gruppen.

Die Formeln erstrecken sich nur auf solche Verbindungen, die mit der chemischen Valenzlehre vereinbar sind. Der im folgenden gebrauchte Hinweis, dass ein Rest durch eine oder mehrere Urethangruppen, O-Atome, S-Atome etc. unterbrochen ist, ist so zu verstehen, dass diese Gruppen jeweils in die Kohlenstoffkette des Restes eingeschoben werden. Diese Gruppen sind damit beidseitig durch C-Atome begrenzt und können nicht endständig sein. $C_1$-Reste können nicht unterbrochen sein. Der Hinweis, dass ein Rest eine Benzolgruppe enthält, bedeutet demgegenüber, dass diese Gruppe auch endständig sein kann, wobei ggf. verbleibende yl-Stellen durch H abgesättigt sind. Unter Kombinationen werden Gruppen verstanden, die sich aus den jeweils angegebenen Bedeutungen zusammensetzen, beispielsweise aus aromatischen und aliphatischen Resten, wie z.B. $-Ph-CH_2-Ph-$, oder aus mehreren aromatischen Resten, wie z.B. -Ph-Ph-, oder aus aromatischen und/oder aliphatischen Resten und anderen der genannten Gruppen, wie z.B. -Ph-O-Ph- (Ph=Phenyl).

Die Verbindungen der Formeln II und III sind bei der radikalischen Polymerisation als Kettenüberträger aktiv und deren Verwendung zur Verringerung der Polymerisationsschrumpfungsspannung von Polymerisaten und zur Verbesserung der Schlagzähigkeit von radikalischen Polymerisaten ist ebenfalls Gegenstand der Erfindung.

In Formel II haben die Variablen die folgenden Bedeutungen:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$ substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;

X -COO-, $-CON(R^3)$- oder entfällt, wobei die Bindung an A über O oder N erfolgt und wobei X entfällt, wenn A ein aromatischer Kohlenwasserstoffrest ist;

B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann,
ein aromatischer $C_6$-$C_{14}$-Kohlenwasserstoffrest, der durch 1 bis 3 Substituenten ausgewählt aus $-CH_3$, $-C_2H_5$, -OH, $-OCH_3$
und/oder $-O-COCH_3$ substituiert sein kann, oder eine Kombination davon, wobei die Kohlenwasserstoffreste endständig eine (Meth)acrylatgruppe tragen können;

Y -COO-, $-CON(R^3)$- oder entfällt, wobei die Bindung an B über O oder N erfolgt und wobei Y entfällt, wenn B ein aromatischer Kohlenwasserstoffrest ist;

$R^{1-3}$ jeweils unabhängig voneinander Wasserstoff, ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OHGruppen substituiert sein kann, oder
ein aromatischer $C_6$-$C_{10}$-Kohlenwasserstoffrest, der durch 1 bis 2 OH-Gruppen substituiert sein kann;

n eine ganze Zahl von 1 bis 3.

Ganz besonders bevorzugt sind Verbindungen der Formel II, in der die Variablen die folgenden Bedeutungen haben

A ein cycloaliphatischer, linearer oder verzweigter aliphatischer $C_1$-$C_8$-Kohlenwasserstoffrest, der durch 1 bis 3 Substituenten ausgewählt aus $-CH_3$, $-C_2H_5$, -OH und/oder - $OCH_3$ substituiert sein kann, der durch 1 bis 2 Urethangruppen, Estergruppen, O und/oder S unterbrochen sein kann, ein aromatischer $C_6$-$C_{10}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;

X -COO-, $-CON(R^3)$- oder entfällt, wobei die Bindung an A über O oder N erfolgt und wobei X entfällt, wenn A ein aromatischer Kohlenwasserstoffrest ist;

B ein cycloaliphatischer, linearer oder verzweigter aliphatischer $C_1$-$C_8$-Kohlenwasserstoffrest, der durch 1 bis 2 Urethangruppen, Estergruppen, O und/oder S unterbrochen sein kann, ein aromatischer $C_6$-$C_{10}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;

Y -COO- oder entfällt, wobei die Bindung an B über O erfolgt und wobei Y entfällt, wenn B ein aromatischer Kohlenwasserstoffrest ist;

$R^{1-3}$ jeweils unabhängig voneinander Wasserstoff, ein cycloaliphatischer, linearer oder verzweigter aliphatischer $C_1$-$C_4$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-Kohlenwasserstoffrest, der durch 1 bis 2 OH-Gruppen substituiert sein kann;

n 1 oder 2.

Insbesondere sind solche Verbindungen der Formel II bevorzugt, in der die Variablen die folgenden Bedeutungen haben:

A ein cycloaliphatischer, linearer oder verzweigter aliphatischer $C_1$-$C_6$-Kohlenwasserstoffrest, der durch $-CH_3$, $-C_2H_5$, -OH oder $-OCH_3$, substituiert sein kann, der durch 1 bis 2 Urethangruppen, Estergruppen, O und/oder S unterbrochen sein kann, ein aromatischer $C_6$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;

X -COO-, $-CON(R^3)$- oder entfällt, wobei die Bindung an A über O oder N erfolgt und wobei X entfällt, wenn A ein aromatischer Kohlenwasserstoffrest ist;

B ein cycloaliphatischer, linearer oder verzweigter aliphatischer $C_1$-$C_6$-Kohlenwasserstoffrest, der durch 1 bis 2

Urethangruppen, Estergruppen, O und/oder S unterbrochen sein kann, ein aromatischer $C_6$-Kohlenwasserstoffrest, der durch 1 bis 3 Substituenten, vorzugsweise $-CH_3$, $-C_2H_5$, -OH, $-OCH_3$ und/oder $-O-COCH_3$, substituiert sein kann, oder eine Kombination davon, wobei die Kohlenwasserstoffreste endständig eine polymerisierbare Gruppe, vorzugsweise eine (Meth)acrylatgruppe tragen können;

Y -COO- oder entfällt, wobei die Bindung an B über O erfolgt und wobei Y vorzugsweise entfällt, wenn B ein aromatischer Kohlenwasserstoffrest ist;

$R^{1-3}$ jeweils unabhängig voneinander Wasserstoff, ein linearer oder verzweigter aliphatischer $C_1$-$C_2$-Kohlenwasserstoffrest, der durch ein Sauerstoffatom unterbrochen sein kann und der durch eine OH-Gruppe substituiert sein kann, vorzugsweise $-CH_3$, $-CH_2-CH_3$ oder $-OCH_3$;

n 1 oder 2.

In Formel III haben die Variablen die folgenden Bedeutungen:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$ substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$, substituiert sein kann, oder eine Kombination davon, wobei die Kohlenwasserstoffreste endständig eine (Meth)acrylatgruppe tragen können;

X -COO-, $-CON(R^3)-$ oder entfällt, wobei die Bindung an A über O oder N erfolgt und wobei X entfällt, wenn A ein aromatischer Kohlenwasserstoffrest ist;

B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$, substituiert sein kann, oder eine Kombination davon, wobei die Kohlenwasserstoffreste endständig eine (Meth)acrylatgruppe tragen können;

Y -COO-, $-CON(R^3)-$ oder entfällt, wobei die Bindung an B über O oder N erfolgt und wobei Y entfällt, wenn B ein aromatischer Kohlenwasserstoffrest ist;

$R^{1-3}$ jeweils unabhängig voneinander Wasserstoff, ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;

n eine ganze Zahl von 1 bis 3.

Ganz besonders bevorzugt sind Verbindungen der Formel III, in der die Variablen die folgenden Bedeutungen haben

A ein cycloaliphatischer, linearer oder verzweigter aliphatischer $C_1$-$C_8$-Kohlenwasserstoffrest, der durch 1 bis 3 Substituenten ausgewählt aus $-CH_3$, $-C_2H_5$, -OH und/oder-$OCH_3$ substituiert sein kann, der durch 1 bis 2 Urethangruppen, Estergruppen, O und/oder S unterbrochen sein kann, ein aromatischer $C_6$-$C_{10}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;

X -COO-, $-CON(R^3)-$ oder entfällt, wobei die Bindung an A über O oder N erfolgt und wobei X entfällt, wenn A ein aromatischer Kohlenwasserstoffrest ist;

B ein cycloaliphatischer, linearer oder verzweigter aliphatischer $C_1$-$C_8$-Kohlenwasserstoffrest, der durch 1 bis 2 Urethangruppen, Estergruppen, O und/oder S unterbrochen sein kann, ein aromatischer $C_6$-$C_{10}$-Kohlenwasserstoffrest, der durch 1 bis 3 Substituenten ausgewählt aus $-CH_3$, $-C_2H_5$, -OH, $-OCH_3$ und/oder $-O-COCH_3$, substituiert sein kann, oder eine Kombination davon, wobei die Kohlenwasserstoffreste endständig eine (Meth)acrylatgruppe tragen können;

Y -COO- oder entfällt, wobei die Bindung an B über O erfolgt und wobei Y entfällt, wenn B ein aromatischer Kohlenwasserstoffrest ist;

$R^{1-3}$ jeweils unabhängig voneinander Wasserstoff, ein cycloaliphatischer, linearer oder verzweigter aliphatischer $C_1$-$C_4$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-Kohlenwasserstoffrest, der durch 1 bis 2 OH-Gruppen substituiert sein kann;

n 1 oder 2.

Insbesondere sind solche Verbindungen der Formel III bevorzugt, in der die Variablen die folgenden Bedeutungen haben:

- A ein cycloaliphatischer, linearer oder verzweigter aliphatischer $C_1$-$C_6$-Kohlenwasserstoffrest, der durch -$CH_3$, -$C_2H_5$, -OH oder -$OCH_3$, substituiert sein kann, der durch 1 bis 2 Urethangruppen, Estergruppen, O und/oder S unterbrochen sein kann, ein aromatischer $C_6$-Kohlenwasserstoffrest, der durch 1 bis 3 Substituenten ausgewählt aus -$CH_3$, -$C_2H_5$, -OH, -$OCH_3$ und/oder -O-$COCH_3$, substituiert sein kann, oder eine Kombination davon, wobei die Kohlenwasserstoffreste endständig eine (Meth)acrylatgruppe tragen können;
- X -COO-, -CON($R^3$)- oder entfällt, wobei die Bindung an A über O oder N erfolgt und wobei X entfällt, wenn A ein aromatischer Kohlenwasserstoffrest ist;
- B ein cycloaliphatischer, linearer oder verzweigter aliphatischer $C_1$-$C_6$-Kohlenwasserstoffrest, der durch 1 bis 2 Urethangruppen, Estergruppen, O und/oder S unterbrochen sein kann, ein aromatischer $C_6$-Kohlenwasserstoffrest, der durch 1 bis 3 Substituenten ausgewählt aus -$CH_3$, -$C_2H_5$, -OH, -$OCH_3$ und/oder -O-$COCH_3$, substituiert sein kann, oder eine Kombination davon, wobei die Kohlenwasserstoffreste endständig eine (Meth)acrylatgruppe tragen können;
- Y -COO- oder entfällt, wobei die Bindung an B über O erfolgt und wobei Y entfällt, wenn B ein aromatischer Kohlenwasserstoffrest ist;
- $R^{1-3}$ jeweils unabhängig voneinander Wasserstoff, ein linearer oder verzweigter aliphatischer $C_1$-$C_2$-Kohlenwasserstoffrest, der durch ein Sauerstoffatom unterbrochen sein kann und der durch eine OH-Gruppe substituiert sein kann, vorzugsweise -$CH_3$, -$CH_2$-$CH_3$ oder -$OCH_3$;
- n 1 oder 2.

Der Einfachheit halber werden hierin die Bezeichnungen für einwertige Reste, wie Phenyl und Naphthyl, auch für mehrwertige Reste mit mehr als einer yl-Stelle verwendet, wobei sich die jeweilige Bedeutung aus den Formeln II und III ergibt. Phenyl (Ph) schließt damit insbesondere Phenylen und Benzol-1,3,5-triyl ein. Naphthyl steht vorzugsweise für einen Naphthalin-2,6-diyl-Rest.

Erfindungsgemäße Verbindungen der Formel II oder III können in Anlehnung an ähnliche bekannte Verbindungen hergestellt werden und sind selbst aber nicht literaturbekannt. Dabei wird im ersten Schritt ein tertiärer Alkohol unter basischen Bedingungen mit einem Halogenid verknüpft. Im zweiten Schritt wird mit einer starken, nicht nukleophilen Base deprotoniert und hydroxymethyliert. Dabei kann die Hydroxymethylierung an der mittelständigen Methylengruppe in Anlehnung an Deguest et al. (G. Deguest, L. Bischoff, C. Fruit, F. Marsais, Org. Lett. 2007, 9, 1165-1167) und Rebein et al. (J. Rehbein, S. Leick, M. Hiersemann, Journal of Organic Chemistry 2009, 74 (4), 1531-1540) durchgeführt werden, wobei Benzotriazol-1-methanol als Reagenz verwendet wird. Der letzte Schritt erfolgt ebenfalls unter basischen Bedingungen, wobei zuerst mesyliert und anschließend eliminiert wird. Bei den Vor- oder Zwischenstufen ist gegebenenfalls die Schutzgruppentechnik anzuwenden:

Schritt 1:

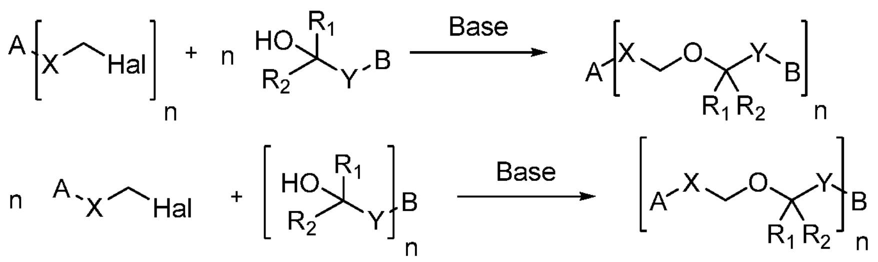


Schritt 2:

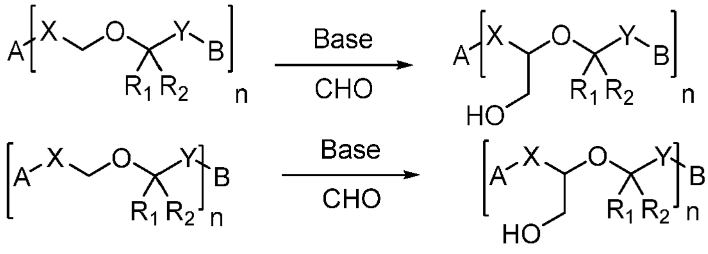

Schritt 3:

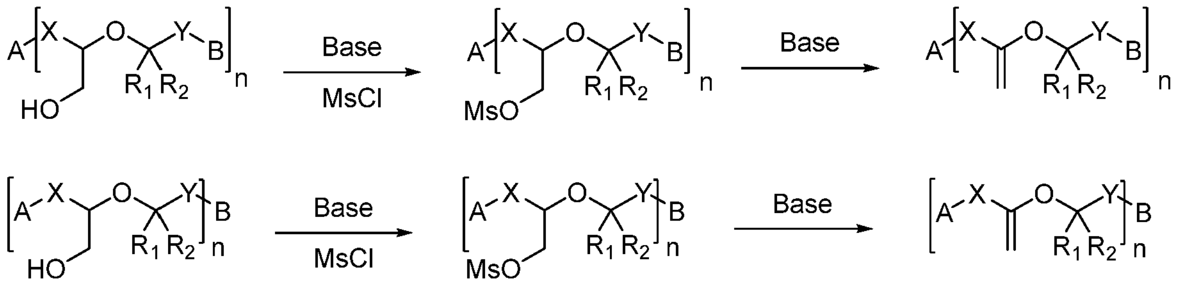


Vorzugsweise lässt sich der besonders bevorzugte 2-((1-Ethoxy-2-methyl-1-oxopropan-2-yl)oxy)acrylsäureethylester (1) dadurch herstellen, dass zunächst Bromessigsäureethylester mit Ethyl-2-hydroxyisobutyrat umgesetzt wird und das Produkt anschließend mit Benzotriazol-1-methanol hydroxymethyliert wird. Nach der Mesylierung zur Einführung einer Abgangsgruppe erfolgt die Eliminierung zum ungesättigten Übertragungsreagenz (1) mit DBU.

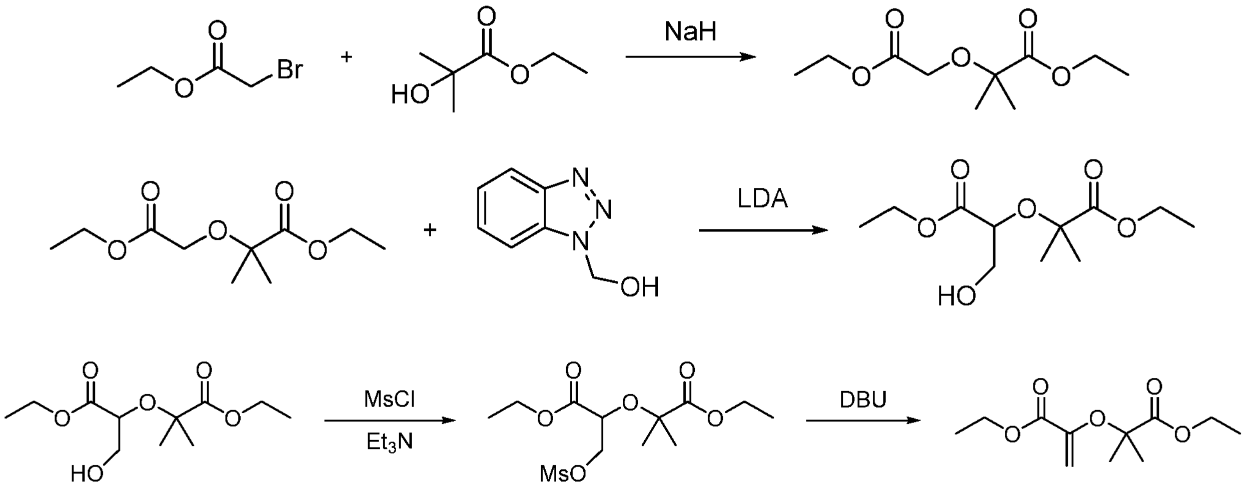


Bevorzugte Beispiele für die erfindungsgemäßen Verbindungen der allgemeinen Formeln II und III sind:

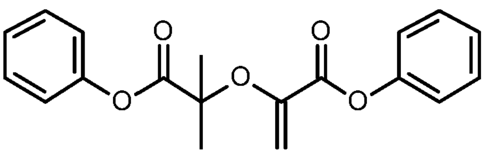

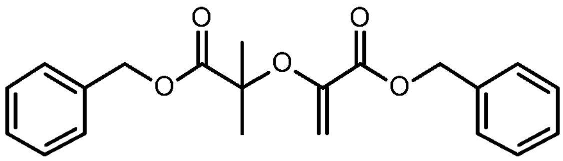

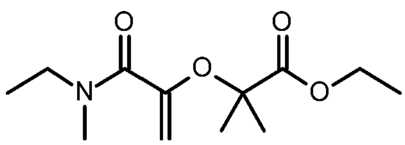

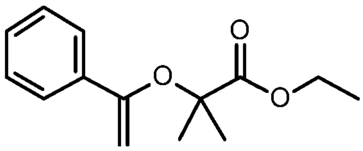

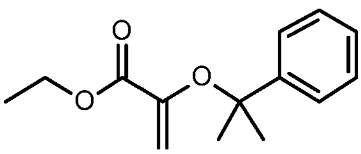

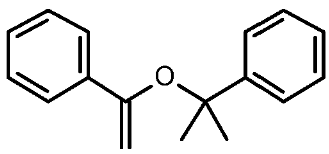

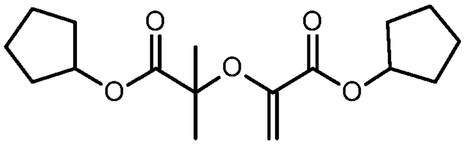

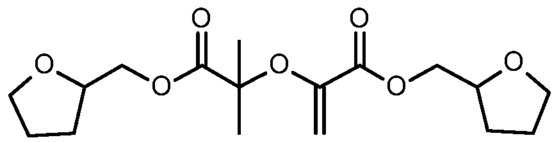

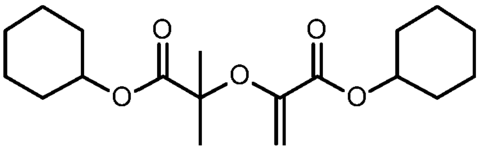

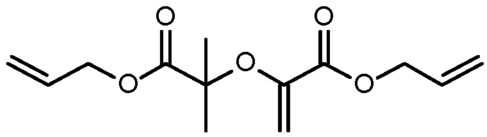

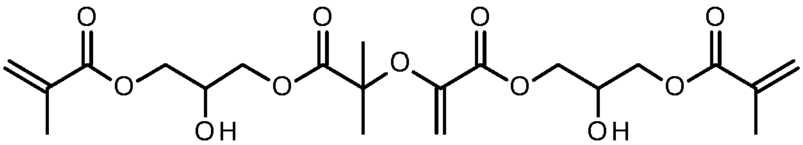

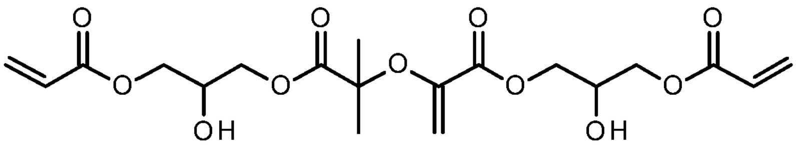

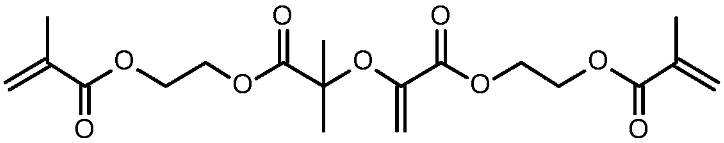

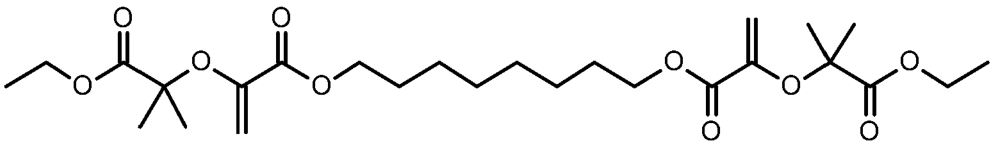

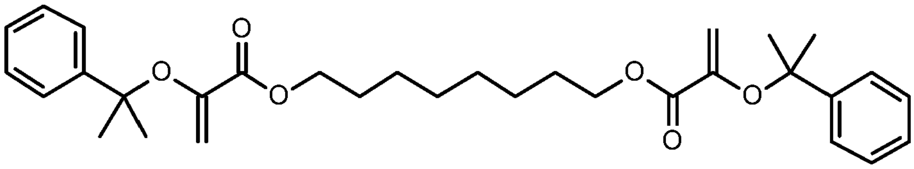

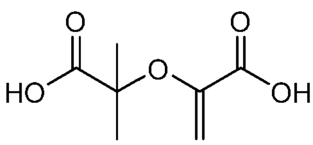

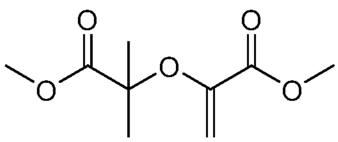

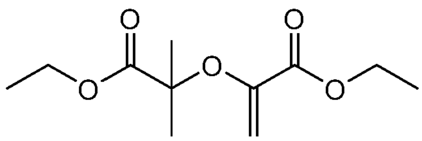

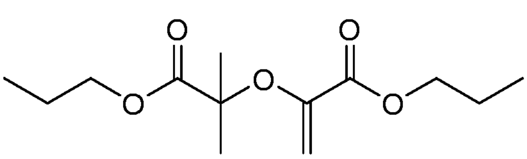

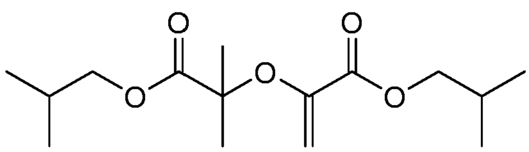

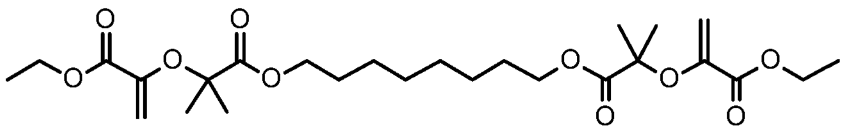

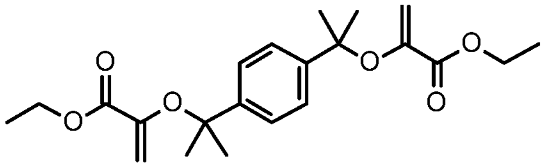

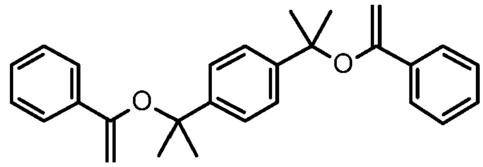

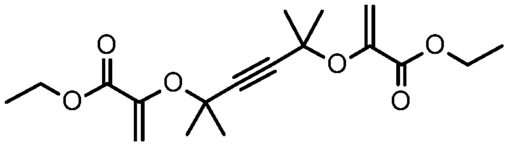

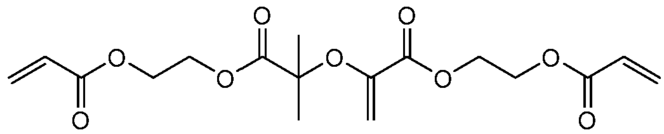

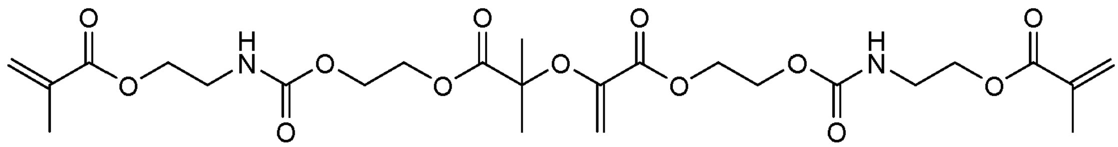

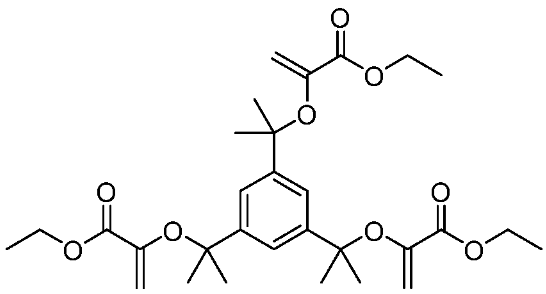

Besonders bevorzugte erfindungsgemäße Verbindungen gemäß der allgemeinen Formeln II und III mit n = 1 sind:

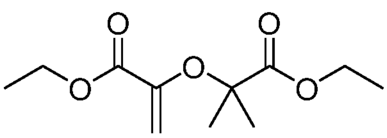

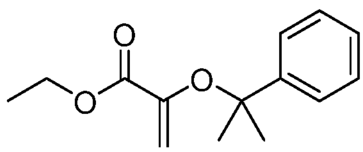

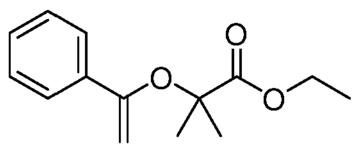

Besonders bevorzugte erfindungsgemäße Verbindungen gemäß der Formel II mit n = 2 sind:

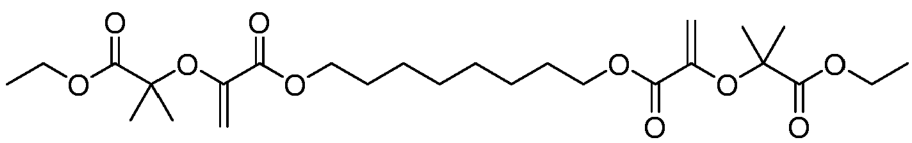

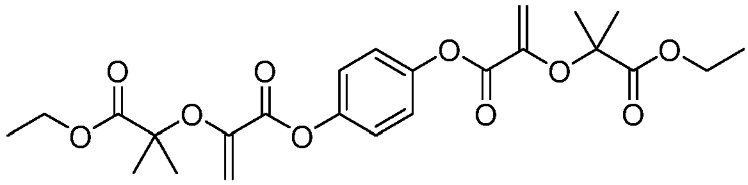

Besonders bevorzugte erfindungsgemäße Verbindungen gemäß der Formel III mit n = 2 sind:

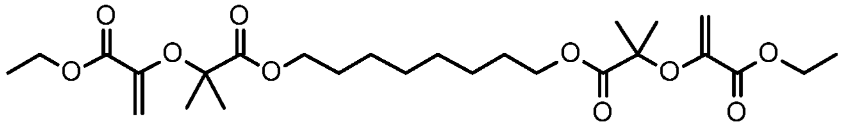

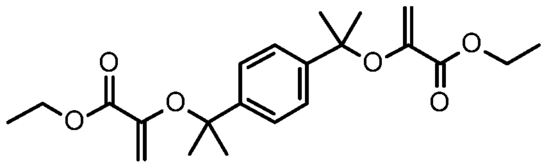

Ferner ist eine besonders bevorzugte erfindungsgemäße Verbindung gemäß der Formel III mit n = 3:

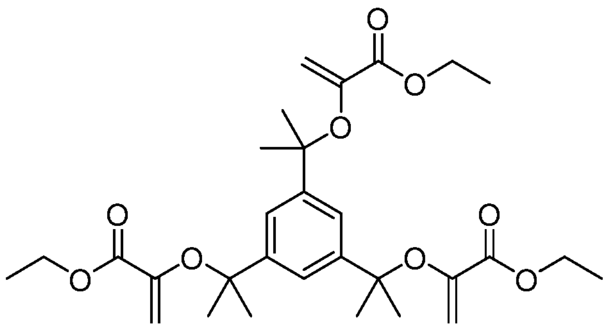

Die erfindungsgemäßen Verbindungen der Formeln II und III lassen sich vorteilhaft als Kettenüberträger zur Kontrolle und Steuerung der Netzwerkstruktur bei der Polymerisation von Mono(meth)acrylaten, multifunktionellen (Meth)acrylaten und deren Mischungen einsetzen. Kettenüberträger werden hierin auch als Übertragungsreagenzien oder Regler bezeichnet. Im Vergleich zu reinen (Meth)acrylaten ergeben sie Polymernetzwerke mit einem engeren Glasübergang, d.h. der Glasübergang findet in einem engeren Temperaturbereich statt, und einer deutlich verringerten Glasübergangstemperatur. Eine verringerte Glasübergangstemperatur hat den Vorteil, dass die Polymeren bei niedrigeren Temperaturen erweicht werden können. Dies gestattet z.B. im Fall von Adhäsiven und Zementen ein gezieltes Lösen der Klebeverbindung (Debonding-on-Demand). Außerdem werden homogenere Polymernetzwerke erhalten, d.h. Netzwerke, die dadurch charakterisiert sind, dass sie eine engere Verteilung der Molmasse zwischen den Vernetzungspunkten aufweisen. Dies hat den Vorteil, dass Kettenspannungen durch Relaxationsprozesse besser abgebaut werden können und dass z.B. im Bereich von Dentalzementen ein schnelleres Debonding-on-Demand (DoD) bewirkt werden kann. Besonders vorteilhaft ist, dass die Verbindungen der Formeln II und III bei der Polymerisation von (Meth)acrylaten die Glasübergangstemperatur der polymerisierten Materialien verringern, ohne die Polymerisationsgeschwindigkeit nennenswert abzusenken.

Darüber hinaus zeichnen sich die erhaltenen Werkstoffe durch eine verbesserte Schlagzähigkeit aus, was z.B. bei stereolithographisch hergestellten Formkörpern oder bei dentalen Prothesen von großem Vorteil ist.

Die Verbindungen der Formeln II und III bewirken bei der vernetzenden Polymerisation von multifunktionellen (Meth)-acrylaten zudem eine deutlich verzögerte Gelbildung und gewährleisten damit eine längere Gelzeit ohne dabei den Umsatz negativ zu beeinflussen, d.h., dass sich das dreidimensionale Polymernetzwerk erst später ausbildet. Die verlängerte Gelzeit wirkt sich vorteilhaft auf die Polymerisationsschrumpfungsspannung aus, weil dadurch innere Spannungen länger durch Fließprozesse ausgeglichen werden können. Dies führt zu deutlich geringeren Schrumpfungsspannungen, was z.B. bei Formteilen mit komplizierten Geometrien oder dentalen Füllungskompositen von großem Vorteil ist.

Die Verbindungen der Formeln II und III können daher zur Verringerung der Glasübergangstemperatur, zur Verringerung der Polymerisationsschrumpfungskraft und/oder der Verbesserung der Schlagzähigkeit von Polymerisaten verwendet werden. Die Polymerisationsschrumpfungskraft wird auch als Polymerisationsschrumpfungsspannung bezeichnet.

Außerdem zeichnen sich die erfindungsgemäßen Werkstoffe durch eine vorteilhaftes Speichermodul bei Raumtemperatur $G'_{(25°C)}$ und eine verbesserte Bruchdehnung aus, sodass die Werkstoffe mechanische Eigenschaften aufweisen, wie sie bei stereolithographisch hergestellten Formkörpern oder bei dentalen Prothesen von Vorteil sind. Das Speichermodul G' ist proportional zu dem Anteil der Deformationsenergie, der im Material gespeichert wird und nach Entlastung wieder aus dem Material gewonnen werden kann. Die Bruchdehnung ist der zuletzt aufgezeichnete Dehnungswert, bevor ein Spannungsabfall auf weniger als oder gleich 10 % des Festigkeitswerts erfolgt. Sie wird als Größe meist in Prozent (%) angegeben.

Erfindungsgemäß sind solche Werkstoffe bevorzugt, die mindestens einen Initiator für die radikalische Polymerisation enthalten.

Die erfindungsgemäßen Werkstoffe enthalten mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die als radikalisch polymerisierbares Monomer mono- und multifunktionelle (Meth)acrylaten enthalten, eignen sich besonders als Dentalwerkstoffe, wobei für Materialien, die intraoral gehärtet werden, Methacrylate bevorzugt sind.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), 2-(2-Biphenyloxy)-ethylmethacrylat, Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di-(meth)acrylat, wie z.B. 2-[4-(2-(Meth)acryloyloxyethoxy-ethoxy)phenyl]-2-[4-(2-methacryloyloxyethoxy)phenyl]-propan) (SR-348c, Firma Sartomer,; enthält 3 Ethoxygruppen) oder 2,2-Bis[4-(2-(meth)acryloxypropoxy)phenyl]propan, 1,6-Bis-[2-(meth)acryloyloxyethoxycarbonylamino]-2,2,4-trimethylhexan (UD(M)A; (ein Additionsprodukt aus 2-Hydroxyethyl(meth)acrylat und 2,2,4-Trimethylhexamethylendiisocyanat), Di-, Tri- oder Tetraethylenglycoldi(meth)acrylat, Trimethylolpropantri(meth)-acrylat, Pentaerythrittetra(meth)acrylat, sowie Glycerindi- und -tri(meth)acrylat, 1,4-Butandioldi(meth)acrylat, 1,10-Decandioldi(meth)acrylat ($D_3MA$) oder 1,12-Dodecandioldi(meth)-acrylat. Ganz besonders geeignete mono- oder multifunktionelle (Meth)acrylate sind Methyl-, Butyl-, Tetrahydrofurfuryl- oder Isobornyl(meth)acrylat, p-Cumyl-phenoxyethylenglycolmethacrylat (CMP-1E), Bisphenol-A-di(meth)acrylat, SR-348c, UD(M)A; Tri- oder Tetraethylenglycoldi(meth)acrylat, Glycerintri-(meth)acrylat, 1,10-Decandioldi(meth)acrylat ($D_3MA$) oder 1,12-Dodecandioldi(meth)acrylat.

Geeignet sind ausserdem auch thermo- oder photolabile Di-(meth)acrylate, wie z.B. das Additionsprodukt aus 2 mol 2-Acetoacetoxyethylmethacrylat und 1 mol 2,2,4-Trimethyl-hexamethylen-1,6-diisocyanat (thermolabil) oder Methacrylsäure-2-[2-(4-{2-methyl-2-[2-(methacryloyloxy)-ethylcarbamoyl-oxy]-propionyl}-phenoxy)-ethoxycarbonylamino]-ethylester. Mischungen von thermo- oder photolabilen Monomeren, wie z.B. dem Additionsprodukt aus 2 mol 2-Acetoacetoxyethylmethacrylat und 1 mol 2,2,4-Trimethylhexamethylen-1,6-diisocyanat (thermolabil), und Verbindungen der Formeln I und II eignen sich besonders für Werkstoffe mit Debonding-on-Demand-Eigenschaften.

Weiterhin enthalten die erfindungsgemäßen Werkstoffe vorzugsweise mindestens einen Photoinitiator oder thermischen Initiator für die radikalische Polymerisation, besonders bevorzugt einen Photoinitiator, insbesondere einen Photoinitiator, der in einem Wellenlängenbereich von 400 bis 500 nm aktiv ist.

Bevorzugte Photoinitiatoren sind Benzophenon, Benzoin, $\alpha$-Diketone, wie 9,10-Phenanthrenchinon, 1-Phenylpropan-1,2-dion, Diacetyl oder 4,4'-Dichlorbenzil oder deren Derivate, besonders bevorzugt Campherchinon (CC) und 2,2-Dimethoxy-2-phenyl-acetophenon, und Mischungen davon.

Die Photoinitiatoren werden vorzugsweise in Kombination mit Beschleunigern eingesetzt. Als Beschleuniger eignen sich besonders tertiäre Amine, wie z.B. tertiäre aromatische Amine, insbesondere N,N-Dialkyl-aniline, -p-toluidine oder -3,5-xylidine, p-N,N-Dialkylamino-phenylethanol, -benzoesäurederivate, -benzaldehyd, -phenylessigsäureester und -phenylpropionsäureester. Ganz besonders bevorzugt sind $\alpha$-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin.

Außerdem bevorzugte Photoinitiatoren sind Norrish-Typ-I-Photoinitiatoren, bevorzugt Acyl- oder Bisacylphosphinoxide, und besonders bevorzugt Monoacyltrialkyl-, Diacyldialkylgermanium- und Tetraacylgermanium-Verbindungen sowie Tetraacylstanane, wie z.B. Benzoyltrimethylgerman, Dibenzoyldiethylgerman, Bis(4-methoxybenzoyl)diethylgerman (MBDEGe, Ivocerin®), Tetrabenzoylgerman, Tetrakis(2-methylbenzoyl)german, Tetrakis(o-methylbenzoyl)german oder Tetrakis(mesitoyl)stannan. Acyl- und Bisacylgermanium-Verbindungen haben den Vorteil, dass sie sich nach der Bestrahlung entfärben (Bleaching Effekt) und so die Transparenz der ausgehärteten Werkstoffe nicht beeinträchtigen. Außerdem handelt es sich um monomolekulare Photoinitiatoren, d.h. sie benötigen keinen Beschleuniger, um ihre volle Aktivität zu erreichen.

Des Weiteren bevorzugt sind Mischungen der verschiedenen oben genannten Photoinitiatoren, wie z.B. eine Mischung von Bis(4-methoxybenzoyl)diethylgerman oder Tetrakis(o-methylbenzoyl)-german mit Campherchinon und 4-Dimethylaminobenzoesäureethylester.

Die erfindungsgemäßen Werkstoffe können einen oder mehrere weitere Initiatoren für die radikalische Polymerisation enthalten, insbesondere thermische Initiatoren, die für die Heißhärtung geeignet sind, bevorzugt Azoverbindungen, wie z.B. 2,2'-Azobis(isobutyronitril) (AIBN) oder Azobis-(4-cyano-valeriansäure), Peroxide, wie z.B. Dibenzoylperoxid, Dilauroylperoxid, tert-Butylperoctoat, tert-Butylperbenzoat oder Di-(tert-butyl)-peroxid, oder Redox-Initiatorkombinationen, wie z.B. Kombinationen von Benzoylperoxid mit Aminen, wie z.B. N,N-Dimethyl-p-toluidin, N,N-Dihydroxyethyl-p-toluidin, p-Dimethylaminobenzoesäureethylester oder N,N-Dimethyl-sym.-xylidin, Kombinationen von anorganischen Peroxiden, wie z.B. Kalium- und Ammoniumperoxodisulfat, mit Reduktionsmitteln, wie z.B. Sulfit, Hydrogensulfit, Thiosulfat, Sulfinsäuren, Aminen, Endiolen oder Fe-(II)-Salzen, Redoxsysteme bestehend aus organischen Peroxiden bzw. Hydroperoxiden und Reduktionsmitteln, wie z.B. Ascorbinsäure, Barbituraten, Thioharnstoffen oder Sulfinsäuren.

Weiterhin lassen sich Verbindungen von Übergangsmetallen, die mindestens zwei stabile Wertigkeitsstufen aufweisen, als Übergangsmetall-Redoxinitiatoren einsetzen. Das sind vor allem Verbindungen der Elemente Kupfer, Eisen, Vanadium, Nickel oder Kobalt, wobei Kupferverbindungen besonders bevorzugt sind und diese bevorzugt als gut organolösliche Verbindungen, wie z.B. als Acetylacetonat, Naphthenat oder 2-Ethyl-hexanoat eingesetzt werden.

Gemäß einer bevorzugten Ausführungsform enthalten die erfindungsgemäßen Werkstoffe, insbesondere Dentalwerkstoffe, zusätzlich organischen oder vorzugsweise anorganischen partikulären Füllstoff, besonders bevorzugt einen oder mehrere anorganische partikuläre Füllstoffe. Mischungen die Monomere, vorzugsweise multifunktionelle (Meth)acrylate, Mischungen davon oder Mischungen von multifunktionellen und monofunktionellen (Meth)acrylaten, und Füllstoffe enthalten, werden als Komposite bezeichnet.

Bevorzugte anorganische partikuläre Füllstoffe sind Oxide, wie $SiO_2$, $ZrO_2$ und $TiO_2$ oder Mischoxide aus $SiO_2$, $ZrO_2$, ZnO und/oder $TiO_2$ mit einer Partikelgröße von 0,010 bis 15 μm, nanopartikuläre oder mikrofeine Füllstoffe mit einer Partikelgröße von 10 bis 300 nm, wie pyrogene Kieselsäure oder Fällungskieselsäure, sowie Glaspulver, wie Quarz-, Glaskeramik- oder röntgenopake Glaspulver, vorzugsweise Barium- oder Strontiumaluminiumsilikatgläser, mit einer Partikelgröße von 0,01 bis 15 μm, vorzugweise von 0,2 bis 1,5 μm, und röntgenopake Füllstoffe, wie Ytterbiumtrifluorid, Tantal(V)-oxid, Bariumsulfat oder Mischoxide von $SiO_2$ mit Ytterbium(III)-oxid oder Tantal(V)-oxid, mit einer Partikelgröße von 0,2 bis 5 μm. Weiterhin können die erfindungsgemäßen Dentalwerkstoffe faserförmige Füllstoffe, Nanofasern, Whiskers oder Mischungen davon enthalten. Besonders bevorzugte Füllstoffe sind $SiO_2$ und $ZrO_2$, Mischoxide aus $SiO_2$ und $ZrO_2$, pyrogene Kieselsäure sowie Glaspulver von Barium- oder Strontiumaluminiumsilikatgläsern und Ytterbiumtrifluorid. Ganz besonders bevorzugte Füllstoffe sind $SiO_2$, Mischoxide aus $SiO_2$ und $ZrO_2$, pyrogene Kieselsäure sowie Glaspulver von Bariumaluminiumsilikatgläsern und Ytterbiumtrifluorid.

Wenn nicht anders angegeben handelt es sich bei allen Partikelgrößen um gewichtsmittlere Partikelgrößen, bei denen die Partikelgrößenbestimmung mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm in einem Messbereich von 0,1 bis 1000 μm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Die Messung der Partikelgröße unter 100 nm erfolgt vorzugsweise durch Dynamische Lichtstreuung (DLS) von wässrigen Partikeldispersionen mit einem ALV / CGS-3 Compact Goniometer (ALV-Laser Vertriebsgesellschaft, Langen, Deutschland) mit einem He-Ne-Laser mit einer Wellenlänge von 633 nm, bei einem Streuwinkel von 90° bei 25°C.

Die Lichtstreuung nimmt mit abnehmender Partikelgröße ab, jedoch haben Füllstoffe mit geringer Partikelgröße eine größere Verdickungswirkung. Die Füllstoffe werden nach der Partikelgröße unterteilt in Makrofüller und Mikrofüller. Makrofüller werden z.B. durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 15 μm. Als Mikrofüller werden vorzugsweise pyrogenes $SiO_2$ oder Fällungskieselsäure eingesetzt oder auch Mischoxide, z.B. $SiO_2$-$ZrO_2$, die durch hydrolytische Cokondensation von Metallalkoxiden zugänglich sind. Die Mikrofüller weisen vorzugsweise eine mittlere Partikelgröße von 5 bis 100 nm auf.

Die Füllstoffe sind vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von $ZrO_2$ oder $TiO_2$, können auch funktionalisierte saure Phosphate, wie 10-Methacryloyloxydecyldihydrogenphosphat eingesetzt werden.

Der Füllungsgrad richtet sich nach dem gewünschten Anwendungszweck. Kompositzemente haben vorzugsweise einen Füllstoffgehalt von 5 bis 70 Gew.-% und Füllungskomposite einen Füllstoffgehalt von 70 bis 85 Gew.-%.

Die erfindungsgemäßen Zusammensetzungen können gegebenenfalls weitere Additive enthalten, vor allem Stabilisatoren, Farbmittel, antibakterielle Wirkstoffe, fluoridionenabgebende Additive, Treibmittel, optische Aufheller, Weichmacher oder UV-Absorber.

Dabei enthalten die Zusammensetzungen auf der Basis der Verbindungen gemäß der allgemeinen Formel II oder III und ihren Mischungen mit mono- und/oder multifunktionellen (Meth)acrylaten vorzugsweise die folgenden Komponenten:

a) 0,1 bis 30 Gew.-%, bevorzugt 1 bis 25 Gew.-% und besonders bevorzugt 1 bis 15 Gew.-% mindestens einer Verbindung gemäß der allgemeinen Formel II oder III,
b) 0,01 bis 5 Gew.-%, besonders bevorzugt 0,1 bis 3,0 Gew.-% mindestens eines Initiators für die radikalische Polymerisation,
c) 1 bis 90 Gew.-%, bevorzugt 1 bis 80 Gew.-% und besonders bevorzugt 10 bis 80 Gew.-% mindestens eines mono- oder multifunktionellen (Meth)acrylates,
d) 0 bis 85 Gew.-%, bevorzugt 0 bis 80 Gew.-% und besonders bevorzugt 0 bis 75 Gew.-% mindestens eines Füllstoffs.

Dabei enthalten Zusammensetzungen ohne Füllstoff vorzugsweise die folgenden Komponenten:

a) 0,1 bis 30 Gew.-%, bevorzugt 1 bis 25 Gew.-% und besonders bevorzugt 1 bis 15 Gew.-% mindestens einer Verbindung gemäß der allgemeinen Formel II oder III,
b) 0,01 bis 5 Gew.-%, besonders bevorzugt 0,1 bis 3,0 Gew.-% mindestens eines Initiators für die radikalische Polymerisation,
c) 1 bis 90 Gew.-%, bevorzugt 1 bis 80 Gew.-% und besonders bevorzugt 10 bis 80 Gew.-% mindestens eines mono- oder multifunktionellen (Meth)acrylates.

Füllstofffreie Zusammensetzungen eignen sich besonders als Prothesenmaterialien oder zur Herstellung von Formkörpern durch Stereolithographie oder 3D-Druck.

Füllstoffhaltige Zusammensetzungen enthalten vorzugsweise die folgenden Komponenten:

a) 0,1 bis 30 Gew.-%, bevorzugt 1 bis 25 Gew.-% und besonders bevorzugt 1 bis 15 Gew.-% mindestens einer Verbindung gemäß der allgemeinen Formel II oder III,
b) 0,01 bis 5 Gew.-%, besonders bevorzugt 0,1 bis 3,0 Gew.-% mindestens eines Initiators für die radikalische Polymerisation,
c) 1 bis 79,89 Gew.-%, bevorzugt 1 bis 70 Gew.-% und besonders bevorzugt 10 bis 70 Gew.-% mindestens eines mono- oder multifunktionellen (Meth)acrylates,
d) 20 bis 85 Gew.-%, bevorzugt 30 bis 80 Gew.-% und besonders bevorzugt 40 bis 75 Gew.-% mindestens eines Füllstoffs.

Füllstoffhaltige Zusammensetzungen eigenen sich besonders zur Herstellung von Inlays, Onlays, Verblendschalen, dentalen Füllungsmaterialien oder als Kompositmassen für die Stereolithographie oder den 3D-Druck.

Alle Prozentangaben hierin sind in Gewichtsprozent und beziehen sich auf die Gesamtmasse des Werkstoffs, wenn nicht anders angegeben.

Besonders bevorzugt sind Zusammensetzungen, die aus den genannten Stoffen bestehen. Weiterhin sind solche Zusammensetzungen bevorzugt, bei denen die einzelnen Komponenten jeweils aus den oben genannten bevorzugten und besonders bevorzugten Stoffen ausgewählt sind.

Es wurde überraschenderweise gefunden, dass Zusammensetzungen, die die erfindungsgemäßen Vinylether gemäß der Formel II oder III enthalten, nach der Härtung vorteilhafte mechanische Eigenschaften aufweisen, wie z.B. eine verbesserte Schlagzähigkeit, eine geringere Schrumpfungsspannung, ein höheres Speichermodul $G'_{(25°C)}$ und eine höhere Bruchdehnung. Diese Eigenschaften sind besonders bei der stereolithographischen Herstellung von Formkörper oder dentalen Prothesen von Vorteil, weil sie eine hohe Dimensionsstabilität und Bruchfestigkeit gewährleisten.

Hervorzuheben ist die verbesserte Schlagzähigkeit der erfindungsgemäßen Materialien. Die Zusammensetzungen weisen nach der Polymerisation bevorzugt eine Schlagarbeit von 3 bis 300 kJ/$m^2$, besonders bevorzugt von 5 bis 250 kJ/$m^2$ und ganz besonders bevorzugt von 10 bis 200 kJ/$m^2$ auf. Die Ermittlung der Zähigkeitseigenschaften erfolgt mittels der DYNSTAT-Anordnung entsprechend der DIN 53435, wobei die Schlagzähigkeit (Schlagarbeit) von ungekerbten Prüfkörpern in der Schlagbiegeanordnung ermittelt wird. Zu diesem Zweck werden Probestäbe (15 x 10 x 4 mm) aus den polymerisierbaren Zusammensetzungen hergestellt und Dynstat-Impact-Tests unter Verwendung eines

10-kpcm-Hammers (10 J) durchgeführt.

Weiterhin weisen die erfindungsgemäßen Zusammensetzungen vorteilhafterweise eine geringere Schrumpfungsspannung auf. Die Zusammensetzungen haben nach der Polymerisation bevorzugt eine Schrumpfungskraft von 1 bis 60 N, besonders bevorzugt von 3 bis 40 N und ganz besonders bevorzugt von 3 bis 30 N. Die Messung der Schrumpfungskraft erfolgt nach der Methode von Watts (D.C. Watts et al., Dental Materials 19, 1-11 (2003)) unter Verwendung einer Universalprüfmaschine. Dabei wird die Probe zwischen einem Stahlstab, der mit der Messzelle der Prüfmaschine verbunden ist, und einer Glasplatte platziert, die mit dem stationären Teil der Prüfmasche befestigt ist. Die Oberflächen des Stahlstabes und der Glasplatte werden zuvor mit einem Primer behandelt, wodurch es zu einem festen Verbund der Probe mit beiden Substraten kommt. Schließlich wird die Probe bestrahlt, was zum Aufbau einer messbaren Schrumpfungskraft führt.

Des Weiteren zeichnen sich die erfindungsgemäßen Zusammensetzungen durch ein vorteilhaftes Speichermodul bei Raumtemperatur $G'_{(25°C)}$ aus. Die Zusammensetzungen weisen nach der Polymerisation bevorzugt ein Speichermodul bei Raumtemperatur $G'_{(25°C)}$ von 0,1 bis 20 MPa, besonders bevorzugt von 0,5 bis 15 MPa und ganz besonders bevorzugt von 0,5 bis 10 MPa auf. Die Bestimmung des Speichermoduls bei Raumtemperatur $G'_{(25°C)}$ erfolgt dadurch, dass die polymerisierbare Zusammensetzungen in Silikonformen gegossen und in einem Lichtofen (z.B. Modell Lumamat 100, Ivoclar AG) polymerisiert werden (z.B. 10 min Bestrahlung mit einer Intensität von ca. 20 $mW/cm^2$). Die Stäbe werden gewendet und abermals bestrahlt. Die Probestäbchen werden dann geschliffen und auf einem Rheometer MCR301 der Firma Anton Paar mit einem CTD (Convection Temperature Control)-Ofen und einer eingesetzten Festkörpereinspannvorrichtung (SRF12 für rechteckige Querschnitte bis 12 mm) vermessen. Die eingestellte Heizrate beträgt 2°C/min. Alle Proben werden von -100°C auf 200°C aufgeheizt und mit einer konstanten Frequenz von 1 Hz und 0,1 % Auslenkung oszilliert.

Außerdem weisen die erfindungsgemäßen Zusammensetzungen eine verbesserte Bruchdehnung auf. Die Zusammensetzungen zeigen nach der Polymerisation bevorzugt eine Bruchdehnung von 0,5 bis 150 %, besonders bevorzugt von 1 bis 100 % und ganz besonders bevorzugt von 2 bis 80 %. Die Bestimmung der Bruchdehnung) erfolgt durch Zugversuche. Dazu werden Probenstäbe nach ISO 527 aus den polymerisierbaren Zusammensetzungen hergestellt und an einer Zwick Z050-Apparatur der Firma Zwick vermessen. Die Versuche selbst werden mit einer Traversengeschwindigkeit von 5 mm/min durchgeführt.

Ferner zeichnen sich die erfindungsgemäßen Zusammensetzungen durch eine erniedrigte Glasübergangstemperatur in einem schmalen Temperaturbereich aus. Die Zusammensetzungen haben nach der Polymerisation bevorzugt eine Glasübergangstemperatur von 10 bis 200°C, besonders bevorzugt von 20 bis 150°C und ganz besonders bevorzugt von 40 bis 150°C. Die Halbwertsbreite der Verlustfaktorkurve beim Glasübergang (HWB) ist dabei bevorzugt kleiner als 150°C, besonders bevorzugt kleiner als 100°C und ganz besonders bevorzugt kleiner als 60°C. Die Bestimmung der Glasübergangstemperatur $T_G$ und der Halbwertsbreite der Verlustfaktorkurve beim Glasübergang (HWB) erfolgt dadurch, dass die polymerisierbare Zusammensetzungen in Silikonformen gegossen und in einem Lichtofen (z.B. Modell Lumamat 100, Ivoclar AG) polymerisiert werden (z.B. 10 min Bestrahlung mit einer Intensität von ca. 20 $mW/cm^2$). Die Stäbe werden gewendet und abermals belichtet. Die Probestäbchen werden dann geschliffen und auf einem Anton Paar-Rheometer MCR301 mit einem CTD- (Convection Temperature Control) Ofen und einer eingesetzten Festkörpereinspannvorrichtung (SRF12 für rechteckige Querschnitte bis 12 mm) vermessen. Die eingestellte Heizrate beträgt 2°C/min. Alle Proben werden von -100°C auf 200°C aufgeheizt und mit einer konstanten Frequenz von 1 Hz und 0,1 % Auslenkung oszilliert.

Die erfindungsgemäßen Werkstoffe eignen sich besonders als Dentalwerkstoffe, insbesondere als dentale Zemente, Füllungskomposite, Verblendmaterialien, Beschichtungsmaterialien und Adhäsive sowie als Materialien zur Herstellung von Prothesen, künstlichen Zähnen, Inlays, Onlays, Kronen und Brücken.

Die Dentalwerkstoffe eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielweise bei der Herstellung oder Reparatur von Dentalrestaurationen, wie Prothesen, künstlichen Zähnen, Inlays, Onlays, Kronen und Brücken.

Durch Polymerisation der erfindungsgemäßer Zusammensetzungen können Homo- und Copolymerisate erhalten werden. Derartige Polymerisate lassen sich beispielsweise durch spanabhebende Verfahren zu Prothesen oder künstlichen Zähnen verarbeiten. Sie liegen vorzugsweise in Form von zylinderförmigen oder scheibenförmigen Rohlingen vor.

Die erfindungsgemäßen Polymermaterialien eigenen sich außerdem zur Herstellung von Formkörpern für dentale aber auch für nicht dentale Zwecke, die z.B. mittels Gießen, Pressen und insbesondere durch generative Verfahren wie den 3D-Druck hergestellt werden können.

Die erfindungsgemäßen Werkstoffe eigenen sich ferner als medizintechnische Werkstoffe zur Herstellung von Medizinprodukten für die Chirurgie, wie z.B. als Werkstoffe zur Herstellung von Implantaten für Gehörprothesen, Knorpel- oder Knochenersatzteile und für die Augenheilkunde, wie z.B. als Werkstoffe zur Herstellung von Intraokularlinsen.

Außerdem betrifft die Erfindung die Verwendung von Verbindungen der Formel II oder III zur Kontrolle oder

Steuerung der Netzwerkstruktur bei der Polymerisation insbesondere von (Meth)acrylaten.

Die Erfindung wird im Folgenden anhand von Beispielen näher erläutert.

## Ausführungsbeispiele

### Beispiel 1

*Synthese von 2-((1-Ethoxy-2-methyl-1-oxopropan-2-yl)oxy)acryl-säureethylester (1)*

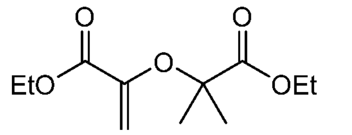

**1**

*1. Stufe: Synthese von 2-(2-Ethoxy-2-oxoethoxy)-2-methylpropansäureethylester (ZP-1)*

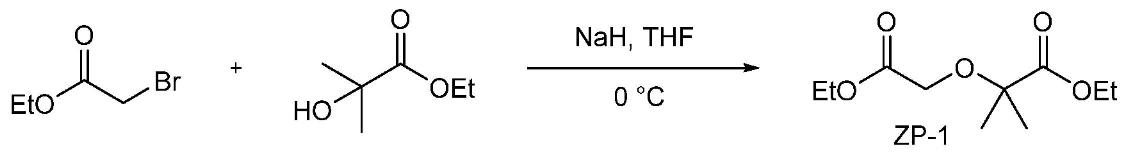


ZP-1

Ein Äquivalent einer 60%igen Natriumhydrid-Mischung (137,5 mmol, 5,48 g) in Mineralöl wurde in 300 ml trockenem Tetrahydrofuran (THF) vorgelegt, auf 0°C gekühlt und die Apparatur mit Argon gespült. Ethyl-2-hydroxyisobutyrat (137,5 mmol, 18,17 g) wurde langsam zugetropft und solange bei 0°C gerührt, bis keine Gasentwicklung mehr zu erkennen war (ca. 1 h). Danach wurde Bromessigsäureethylester (137,5 mmol, 22,95 g) zugetropft und über Nacht bei Raumtemperatur gerührt. Die Reaktion wurde durch Zugabe von 100 ml gesättigter $NH_4Cl$-Lösung abgebrochen, die wässrige Phase dreimal mit 150 ml Ethylacetat extrahiert und die vereinte organische Phase über wasserfreiem $Na_2SO_4$ getrocknet. Das Lösungsmittel wurde abgezogen und das Rohprodukt wurde mittels Hochvakuumdestillation gereinigt (Siedepunkt: 70°C bei 0,07 mbar). Das Produkt ZP1 konnte in einer Ausbeute von 19,4 g (65 % der theoretischen Ausbeute) als farblose Flüssigkeit erhalten werden.

Rf-Wert: 0,5 (Petrolether (PE):Ethylacetat (EE) 5:1).

$^1$H-NMR: (400 MHz, $CDCl_3$) $\delta$ (ppm): 4,20 (4H, m, $-CH_2-CH_3$), 4,10 (2H, s, $-CH_2-O-$), 1,47 (6H, s, $-C-CH_3$), 1,27 (6H, t, $-CH_2-CH_3$, J = 7,1 Hz).

$^{13}$C-NMR: (100 MHz, $CDCl_3$) $\delta$ (ppm): 174,0 (C=O), 170,4 (C=O), 78,5 (C4), 63,3 (C2), 61,3 (C2), 61,0 (C2), 24,6 (C1), 14,3 (C1).

*2. Stufe: Synthese von 2-((1-Ethoxy-2-methyl-1-oxopropan-2-yl)oxy)-3-hydroxypropansäureethylester (ZP-2)*

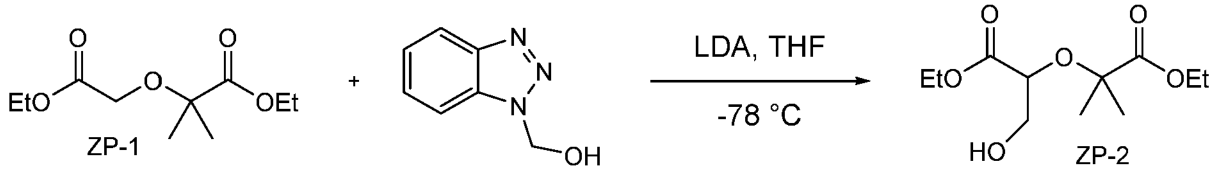


ZP-1 ZP-2

Die Reaktion wurde in einem 1000 ml Dreihalskoben unter Argon-Atmosphäre durchgeführt. 3 Äquivalente n-Butyllithium (137,5 mmol, 55 ml 2,5 M in Hexan) wurden in 250 ml trockenem Tetrahydrofuran (THF) vorgelegt und auf -10°C gekühlt. 3,5 Äquivalente trockenes Diisopropylamin (160,3 mmol, 16,2 g) wurden zugetropft und die Reaktionslösung wurde für 30 min bei 0°C gerührt. Danach wurde die Reaktionslösung auf -78°C gekühlt und eine Lösung von

(2-(2-Ethoxy-2-oxoethoxy)-2-methylpropansäureethylester aus Stufe 1 (14,5 mmol, 3,59 g) in 60 ml THF langsam zugetropft. Schließlich wurde die Reaktionslösung für 1 h bei -78°C gerührt, Benzotriazol-1-methanol (91,6 mmol, 13,7 g) gelöst in 250 ml THF wurde langsam zugetropft und die Reaktionslösung danach für 2 h bei -78°C gerührt. Die Reaktion wurde durch Zugabe von 150 ml gesättigter $NH_4Cl$-Lösung abgebrochen und die wässrige Phase dreimal mit je 200 ml Diethylether extrahiert. Die vereinigte organische Phase wurde mit je 150 ml 4N NaOH und gesättigter Kochsalzlösung gewaschen, wobei der Waschschritt mit 4N NaOH so rasch wie möglich durchgeführt wurde. Die organische Phase wurde über wasserfreiem $Na_2SO_4$ getrocknet und das Rohprodukt als gelbes Öl in einer Ausbeute von 7,70 g (77% der theoretischen Ausbeute) nach Abdampfen des Lösungsmittels erhalten. Das Rohprodukt wurde mittels Säulenchromatographie (Petrolether (PE) :Ethylacetat (EE) 5:1 → 3:2) gereinigt und das Zwischenprodukt ZP-2 in einer Ausbeute von 3,59 g (36% der theoretischen Ausbeute) erhalten.

Rf-Wert: 0,3 (PE:EE 3:1).

$^1$H-NMR: (400 MHz, $CDCl_3$) δ (ppm): 4,20 (5H, m, $-CH_2-CH_3$, O-CH-), 3,84 (2H, m, $-CH_2$-OH), 1,51 (3H, s, -C-$CH_3$), 1,44 (3H, s, -C-$CH_3$), 1,27 (6H, t, $-CH_2-CH_3$, J= 7,1 Hz).

$^{13}$C-NMR: (100 MHz, $CDCl_3$) δ (ppm): 175,4 (C=O), 171,4 (C=O), 79,0 (C4), 76,2 (C3), 64,3 (C2), 61,8 (C2), 61,3 (C2), 27,3 (C1), 22,7 (C1), 14,3 (C1), 14,3 (C1).

*3. Stufe: Synthese von 2-((1-Ethoxy-2-methyl-1-oxopropan-2-yl)oxy)acryl-säureethylester (1)*

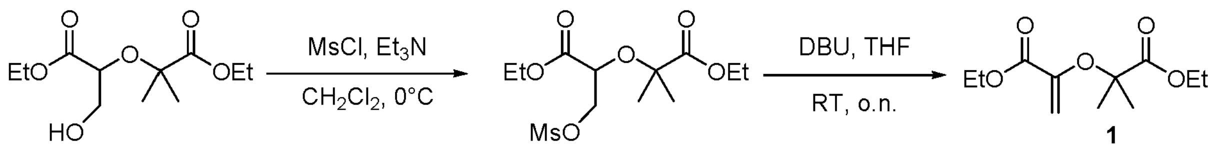


ZP-2 (14,5 mmol, 3,59 g) wurde unter Argon-Atmosphäre in 45 ml trockenem Dichlormethan vorgelegt, die Lösung wurde auf 0°C gekühlt und 1,3 Äquivalente Triethylamin (18,8 mmol, 1,90 g) wurden zugesetzt. Dann wurden 1,2 Äquivalente Mesylchlorid (17,4 mmol, 1,99 g) zugetropft und die Lösung bei Raumtemperatur 1 h gerührt. Die Reaktion wurde durch Zugabe von 30 ml gesättigter $NaHCO_3$-Lösung abgebrochen und die wässrige Lösung dreimal mit 30 ml Dichlormethan extrahiert. Nach Entfernen des Lösungsmittels wurde das Rohprodukt in trockenem Tetrahydrofuran (THF) gelöst, unter Argon-Atmosphäre gesetzt und auf 0°C gekühlt. 3 Äquivalente Diazabicycloundecen (DBU, 43,4 mmol, 8,60 g) wurden zugesetzt und die Reaktionslösung wurde bei Raumtemperatur über Nacht gerührt. Die Reaktion wurde durch Zugabe von 20 ml Wasser abgebrochen, die wässrige Phase dreimal mit je 20 ml Dichlormethan extrahiert und über wasserfreiem $Na_2SO_4$ getrocknet. Das Lösungsmittel wurde abgezogen und ergab das Rohprodukt als farbloses Öl. Das überschüssige DBU konnte mittels Säulenchromatographie entfernt werden (Laufmittel Petrolether (PE) :Ethylacetat (EE) 10:1) und das Reinprodukt 1 wurde in einer Ausbeute von 2,32 g (70 % der theoretischen Ausbeute) erhalten.

Rf-Wert: 0,4 (PE:EE 10:1)

$^1$H-NMR: (400 MHz, $CDCl_3$) δ (ppm) : 5,53 (1H, d, C=$CH_2$, J = 2,7 Hz), 4,55 (1H, d, C=$CH_2$, J = 2,7 Hz), 4,22 (4H, qq, $-CH_2-CH_3$), 1,59 (6H, s, -C-$CH_3$), 1,29 (6H, 2 t, $-CH_2-CH_3$).

$^{13}$C-NMR: (100 MHz, $CDCl_3$) δ (ppm): 173,4 (C=O), 163,7 (C=O), 147,9 (C4), 100,3 (C2), 79,7 (C4), 61,6 (C2) , 24,6 (C1), 14,3 (C1), 14,3 (C1).

**Beispiel 2**

*Synthese von 2,2-Dimethyl-4-methylenglutarsäuredimethylester (2) (Referenzverbindung)*

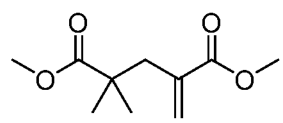

**2**

Die Synthese der Referenzverbindung (2) wurde analog zu Moad et al. (Moad, C. L.; Moad, G.; Rizzardo, E.;

Thang, S. H., Chain Transfer Activity of ω-Unsaturated Methyl Methacrylate Oligomers. Macromolecules 1996, 29 (24), 7717-7726) durchgeführt.

**Beispiel 3**

*Synthese von Bis (2-hydroxy-3- (methacryloyloxy)propyl) -2,2-dimethyl-4-methylenepentandioat (3) (Referenzverbindung)*

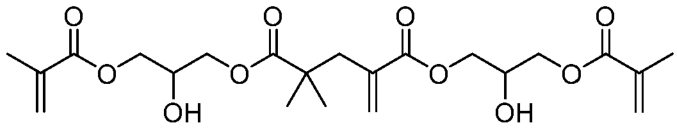

**3**

Die Synthese von Verbindung (3) wurde analog zu WO 2012/112350 A2 durchgeführt.

**Beispiel 4**

*Synthese von 2-({1-[2-(Methacryloyloxy)ethoxy]-2-methyl-1-oxo-propan-2-yl}oxy)acrylsäure-2-(methacryloyloxy)ethylester (4)*

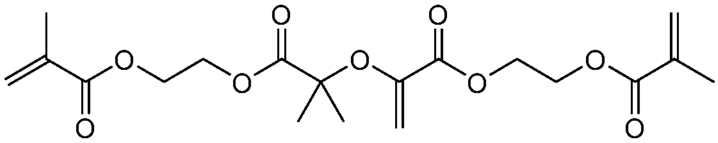

**4**

*1. Stufe: 2-[(2-Carboxypropan-2-yl)oxy]acrylsäure (ZP-3)*

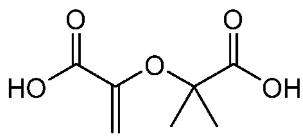

2-[(1-Ethoxy-2-methyl-1-oxopropan-2-yl)oxy]acrylsäureethyl-ester 1 (3,06 g; 13,3 mmol) wurde zu einer Lösung von Kaliumhydroxid (2,09 g; 37,2 mmol) in Wasser (30 ml) zugegeben und das Gemisch wurde 24 h auf 90°C erhitzt. Nach dem Abkühlen wurde die Reaktionslösung mit Salzsäure (2N) angesäuert (pH = 1) und mit *tert.*-Butylmethylether (4x 50 ml) extrahiert. Die vereinigten organischen Phasen wurden über wasserfreiem Natriumsulfat getrocknet, filtriert und am Rotationsverdampfer eingeengt. Man erhielt 2,29 g ZP-3 (13,1 mmol; 99 % Ausbeute) als gelbes Öl.

$^1$H-NMR ($CDCl_3$, 400 MHz) : δ = 10, 17 (s, 2H; OH), 5, 83 (d, 1H; J = 2,5 Hz; =CH), 4, 93 (d, 1H; *J* = 2,5 Hz; =CH), 1,62 (s, 6H; $CH_3$).

$^{13}$C-NMR ($CDCl_3$, 100.6 MHz) : δ = 176, 0 (C=O), 167,3 (C=O), 146,2 (=C), 105,4 (=$CH_2$), 80, 3 (C), 24, 2 ($CH_3$).

*2. Stufe: 2-({1-[2-(Methacryloyloxy)ethoxy]-2-methyl-1-oxo-propan-2-yl}oxy)acrylsäure-2-(methacryloyloxy)-ethylester (4)*

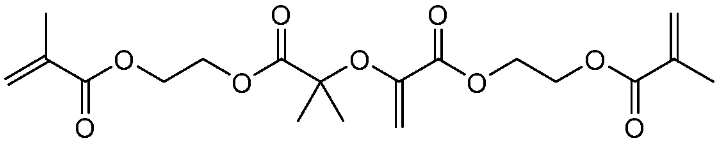

**4**

Eine Lösung von 2-[(2-Carboxypropan-2-yl)oxy]acrylsäure ZP-3 (2,19 g; 12,6 mmol), 2-Hydroxyethylmethacrylat (3,44 g, 26,4 mmol), *N,N*-Dimethylaminopyridin (0,24 g; 2,0 mmol) und 2,6-Di-*tert*.-butyl-4-methylphenol (10 mg) in Dichlormethan (100 ml) wurde auf 0°C abgekühlt und *N*-(3-Dimethylaminopropyl)-N'-ethylcarbodiimid Hydrochlorid (5,30 g; 27,7 mmol) wurde portionsweise zugegeben. Das Reaktionsgemisch wurde 2 h unter Eiskühlung und anschließend bei Umgebungstemperatur gerührt. Nach 20 h wurde die Lösung mit n-Heptan (100 ml) verdünnt und über eine Schicht Kieselgel filtriert. Das Filtrat wurde am Rotationsverdampfer eingeengt. Das Rohprodukt wurde mittels Säulenchromatographie gereinigt ($SiO_2$, *n*-Heptan/Ethylacetat 3:1; $R_f$ = 0,3). Man erhielt 2,39 g von 4 (6,0 mmol; 48 % Ausbeute) als gelbliches Öl.

$^1$H-NMR ($CDCl_3$, 400 MHz) : $\delta$ = 6,13 (m, 1H; =CH), 6,10 (m, 1H; =CH), 5,59 (m, 2H; =CH), 5,56 (d, 1H; *J* = 2,5 Hz; =CH), 4,64 (d, 1H; *J* = 2,5 Hz; =CH), 4.40 (m, 8H; $CH_2$), 1,95 (m, 3H; $CH_3$), 1,93 (m, 3H; $CH_3$), 1,59 (s, 6H; $CH_3$).

$^{13}$C-NMR ($CDCl_3$, 100.6 MHz) : $\delta$ = 172,8 (C=O), 166,8 (C=O), 166,7 (C=O), 162,9 (C=O), 147,0 (=C), 135,7 (=C), 135,7 (=C), 125,9 (=$CH_2$), 125,9 (=$CH_2$), 101,3 (=$CH_2$), 79,5 (C), 62,8 ($CH_2$), 62,7 ($CH_2$), 61, 9 ($CH_2$), 61, 9 ($CH_2$), 24, 2 ($CH_3$), 18, 0 ($CH_3$), 18,0 ($CH_3$).

**Beispiel 5**

*Reaktivitätsmessungen mit Benzylmethacrylat*

Um die Polymerisationsreaktivität und Regelungsfähigkeit der erfindungsgemässen Verbindungen zu untersuchen, wurden Formulierungen mit dem monofunktionellem Monomer Benzylmethacrylat (BMA) und 20 Doppelbindungsprozent (DB%) des Übertragungsreagenzes bezogen auf die Gesamtmenge an Doppelbindungen in der Formulierung hergestellt. Als Übertragungsreagenz wurde dabei (1) gemäß Beispiel 1 mit (2) aus Beispiel 2 verglichen. Allen Formulierungen wurde zusätzlich 1 mol% Bis(4-methoxybenzoyl) - diethylgermanium (BMDGe) als Photoinitiator zugesetzt. Für die Polymerisation wurde ein Differential-Scanning-Kalorimeter (DSC) Netzsch DSC 204 F1 Phoenix® (Netzsch-Gerätebau GmbH) mit Autosampler verwendet. Die Messungen wurden isotherm bei 25°C unter Stickstoffatmosphäre durchgeführt. 10 $\pm$ 1 mg Probenformulierung wurden in ein Aluminium-DSC-Schälchen eingewogen, das mittels Autosampler in der DSC Kammer platziert wurde. Die Probe wurde 4 min lang mit Stickstoff gespült (20 ml/min) und anschließend 5 min lang mittels gefiltertem UV-Licht (Breitband Hg-Lichtquelle EXFO Omnicure 2000 der Firma Excelitas) im sichtbaren Bereich von 400-500 nm mit einer Intensität von 1 W/cm$^2$ am Strahlenausgang der Lampe bestrahlt. Zur Beurteilung der Reaktivität wurden die Zeit bis zum Erreichen von 95 % des maximalen Umsatzes ($t_{95}$) und die Zeit bis zum Erreichen der maximalen Polymerisationsgeschwindigkeit ($t_{max}$) herangezogen.

Die polymerisierten Proben wurden in Tetrahydrofuran (THF) gelöst und mit einer Gelpermeationschromatographie (GPC) Waters GPC mit drei in Serie geschalteten Säulen (Styragel HR 0.5, Styragel HR 3 und Styragel HR 4) und einem Waters 2410 RI Detektor in einem Säulenofen bei 40°C und mit einer Flussrate von 1,0 ml/min analysiert. Zur Kalibrierung wurden Polystyrolstandards verwendet. Das Verhältnis zwischen dem zahlenmittleren Molekulargewicht des mit Übertragungsreagenz geregelten Polymers und jenem von reinem Poly-BMA ($Mn_{mod}/Mn_{BHA}$) zeigt, wie stark das durchschnittliche Molekulargewicht durch das Übertragungsreagenz gesenkt wird. Der Polydispersitätsindex (PDI) gibt die Molmassenverteilung der erhaltenen Polymere an. Für eine erfolgreiche Regelung ist eine signifikante Verringerung des Molekulargewichts, d.h. ein niedriger Wert des Verhältnisses $Mn_{mod}/Mn_{BMA}$, und des PDI, bei gleichzeitig hoher Polymerisationsgeschwindigkeit, d.h. relativ niedrigen Werten für $t_{95}$ und $t_{max}$, anzustreben. In Tabelle 1 handelt es sich bei Vergleichsversuch V1 um die Polymerisation von reinem monofunktionellen Methacrylat BMA ohne Übertragungsreagenz. In Versuch B1 wurde eine Mischung von BMA und erfindungsgemäßen Übertragungsreagenz (1) verwendet, während bei Vergleichsversuch V2 eine Mischung aus BMA und dem Referenzübertragungsreagenz (2) untersucht wurde.

***Tabelle 1: Ergebnisse der Reaktivitätsmessungen mit* dem *Übertragungsreagenz (1) und der Referenzverbindung (2)***

| Regler | Beispiel | $t_{95}$ [S] | $t_{max}$ [s] | $DBC_{BMA}$ // $DBC_{Regler}$ [%] // [%] | $M_n$ [kDa] | $Mn_{mod}/Mn_{BMA}$ | PDI |
|---|---|---|---|---|---|---|---|
| - | V1 | 98 | 18, 9 | 58 // - | 3,6 | - | 3, 4 |

(fortgesetzt)

| Regler | Beispiel | $t_{95}$ [S] | $t_{max}$ [s] | $DBC_{BMA}$ // $DBC_{Regler}$ [%] // [%] | $M_n$ [kDa] | $Mn_{mod}/Mn_{BMA}$ | PDI |
|---|---|---|---|---|---|---|---|
| 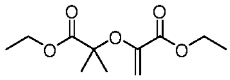 (1) | B1 | 127 | 17,1 | 50 // 30 | 1,2 | 0,33 | 1,9 |
| 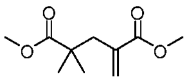 (2) | V2 | 125 | 16, 9 | 24 // 12 | 1,4 | 0,39 | 2,3 |

Tabelle 1 zeigt, dass bei erfindungsgemäßem Einsatz von Verbindung (1) als Übertragungsreagenz sowohl $t_{95}$ als auch $t_{max}$ im Vergleich zu reinem BMA in Vergleichsbeispiel 1 erhöht werden, wobei auch Verbindung (2) in Vergleichsbeispiel 2 diese Retardierung in ähnlichem Maß zeigt. Betrachtet man jedoch den Doppelbindungsumsatz (DBC) von BMA und des Übertragungsreagenzes, erkennt man sehr deutlich, dass die Mischung mit Übertragungsreagenz (1) signifikant höhere Umsätze als die Mischung mit Referenzverbindung (2) aufweist und somit die Anforderungen an ein Übertragungsreagenz besser erfüllt. Dies wird auch bei Betrachtung des Verhältnisses des Zahlenmittels $M_n$ zwischen reguliertem und unreguliertem Poly(BMA) deutlich. Während bei V2 mit der Referenzverbindung (2) das niedrige Molekulargewicht durch den sehr geringen Umsatz erklärt werden kann, erhält man bei B1 mit Übertragungsreagenz (1) sowohl niedriges Molekulargewicht, als auch annehmbaren Doppelbindungsumsatz.

**Beispiel 6**

*Reaktivitätsmessungen für Polymermaterialien mit Dimethacrylaten*

Um die Reaktivität der erfindungsgemäßen Verbindung (1) ebenfalls in einer anwendungsnäheren Polymermatrix zu untersuchen, wurden Photo-DSC-Messungen für Polymermaterialien mit difunktionellen Methacrylaten durchgeführt. Die Untersuchungen wurden unter den gleichen Bedingungen wie in Beispiel 5 mit einem Differential-Scanning-Kalorimeter (DSC) Netzsch DSC 204 F1 Phoenix® (Netzsch-Gerätebau GmbH) mit Autosampler durchgeführt. Als Polymermatrix ohne Übertragungsreagenz wurde ein äquimolares Gemisch der kommerziell erhältlichen Dimethacrylate Urethandimethacrylat (UDMA, Isomerengemisch; CAS: 72869-86-4) und 1,10-Decandioldimethacrylat ($D_3MA$) (Harzmischung, 2M) eingesetzt. Als Übertragungsreagenz wurden dabei erneut die erfindungsgemäße Verbindung (1) und die Referenzverbindung (2) verwendet, wobei zusätzlich auch die aus WO 2012/112350 A2 bekannte Verbindung (3) untersucht wurde. Der Basismischung wurden 20 Doppelbindungsprozent (DB%) des jeweiligen Übertragungsreagenzes (1) und (2) bezogen auf die Gesamtmenge an Doppelbindungen in der Formulierung zugesetzt. Zusätzlich wurde eine Mischung der Basismatrix mit 0,20 molaren Äquivalenten der Verbindung (3) hergestellt.

***Tabelle 2: Ergebnisse der Reaktivitätsmessungen mit dem Übertragungsreagenz (1) und den Referenzverbindungen (2) und (3)***

| Verbindung | Beispiel | $t_{95}$ [S] | $t_{max}$ [s] | DBC [%] |
|---|---|---|---|---|
| *Kein Übertragungsreagenz (nur UDMA/$D_3MA$)* | V3 | 45, 2 | 4,2 | 67 |
| 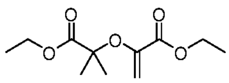 (1) | B2 | 105, 0 | 14,5 | 87 |
| 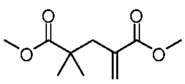 (2) | V4 | 129 | 15,4 | 39 |

(fortgesetzt)

| Verbindung | Beispiel | $t_{95}$ [S] | $t_{max}$ [s] | DBC [%] |
|---|---|---|---|---|
| 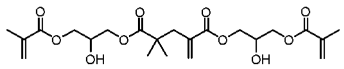 3 (3) | V5 | 85, 2 | 14,3 | 67 |

Aus Tabelle 2 geht hervor, dass die Verbindung 1 bei erfindungsgemäßer Verwendung als Übertragungsreagenz (Versuch B2), zwar die Zeit bis zum Erreichen der maximalen Polymerisationsgeschwindigkeit ($t_{max}$) als auch die Zeit bis zum Erreichen von 95-%-Umsatz ($t_{95}$) im Vergleich zum Vergleichsversuch V3 ohne Übertragungsreagenz erhöht, jedoch auch eine Erhöhung des Doppelbindungsumsatzes auf 87% (67 % bei V3) bewirkt. Bei Vergleichsversuch V4 mit Referenzverbindung (2) erkennt man ebenfalls den signifikanten Anstieg von $t_{max}$ und $t_{95}$, der jedoch noch deutlicher als bei B2 ausfällt. Allerdings wird hier auch der Doppelbindungsumsatz stark auf nur noch 39% erniedrigt. Somit wirkt Referenzverbindung (2) in der Polymermatrix bloß retardierend, während Verbindung (1) das System zwar etwas verlangsamt, jedoch effektiv reguliert und den Umsatz erhöht. Der Zusatz von Hybridmonomer 3 in Vergleichsversuch V5 führt zwar zu einer deutlichen Retardierung des Systems, dennoch aber zu keiner Erhöhung des Doppelbindungsumsatzes, im Vergleich mit V3. Folglich ist anzunehmen, dass die Referenzverbindung (3) lediglich wie ein Comonomer reagiert, anstatt die Polymerisation durch Kettenübertragung zu regeln.

**Beispiel 7**

*Herstellung und Charakterisierung von Dimethacrylat-Photopolymerisaten mit einem RT-NIR-Photorheometer*

Ähnlich wie in den vorangegangenen Beispielen wurden Polymermaterialien, bestehend aus einem äquimolaren Gemisch von UDMA und $D_3MA$ (Vergleichsbeispiel 6) und dem jeweiligen Übertragungsreagenz hergestellt. Der UDMA-$D_3MA$-Basismatrix wurden jeweils 20 Doppelbindungsprozent (DB%) der Verbindung (1) (Beispiel 3), der Referenzverbindung (2) (Vergleichsbeispiel 7) und 0,2 molare Äquivalente der Verbindung (3) (Vergleichsbeispiel 8) bezogen auf die Gesamtmenge an Doppelbindungen in der Formulierung zugesetzt. Allen Formulierungen wurde zusätzlich 1 Gew.-% Bis(4-methoxybenzoyl)diethylgerman (BMDGe, Ivocerin®) als Photoinitiator hinzugefügt. Zur Überprüfung der Photoreaktivität wurden die hergestellten Formulierungen mit einem Echtzeit-Nahinfrarot (RT-NIR) Photorheometer MCR302 WESP der Firma Anton Paar vermessen, das zur Umsatzkontrolle mit einem Bruker Vertex-80-IR-Spektrometer gekoppelt wurde. Es wurde ein PP-25-Messsystem verwendet, und der Messspalt wurde auf 0,2 mm eingestellt. Vor und während der Aushärtung (10 mW/$cm^2$ auf Probenoberfläche; 400-500 nm; Omnicure 2000 UV-Lampe) wurden Speicher- und Verlustmodul der Proben im Oszillationsmodus (1 % Auslenkung, 1 Hz) gemessen. Gleichzeitig wurden während der Messung IR-Spektren der Probe mit einer Frequenz von ~5 Hz aufgezeichnet. Als Maß für die Photoreaktivität wurde das Erreichen des Gelpunkts (Schnittpunkt von Speicher- und Verlustmodul) und die Dauer bis zum Erreichen von 95 % des finalen Speichermoduls ($t_{95\%}$) herangezogen. Zusätzlich wurden der Umsatz am Gelpunkt ($DBC_g$), der Gesamtumsatz (DBC) und die photopolymerisationsinduzierte Schrumpfungsspannung ($F_S$) ermittelt. Die erzielten Ergebnisse sind in Tabelle 3 zusammengefasst.

***Tabelle 3: RT-NIR-Photorheometrie***

| Beispiel | Verbindung | Gelpunkt [s] | $DBC_g$ [%] | DBC [%] | $t_{95\%}$ [S] | $F_S$ [N] |
|---|---|---|---|---|---|---|
| V6 | - | 1,3 | 17 | 79 | 41 | -41 |
| B3 | (1)[a] | 15, 3 | 31 | 91 | 202 | -26 |
| V7 | (2)b | 54,3 | 17 | 43 | 279 | -15 |
| V8 | (3)[c] | 8,5 | 10 | 65 | 93 | -28 |
| a = 2-((1-Ethoxy-2-methyl-1-oxopropan-2-yl)oxy)acryl-säureethylester<br>b = 2,2-Dimethyl-4-methylenglutarsäuredimethylester<br>c = Bis(2-hydroxy-3-(methacryloyloxy)propyl)-2,2-dimethyl-4-mehylenepentandioat | | | | | | |

Die Ergebnisse in Tabelle 3 zeigen, dass der Zusatz von beiden Übertragungsreagenzien (1) und (2) jeweils zu einer Verzögerung des Gelpunkts führt. Zusätzlich wurde bei der erfindungsgemäßen Verbindung (1) sowohl der Umsatz am Gelpunkt (31 %) als auch der finale Doppelbindungsumsatz (91 %) deutlich erhöht, während Referenzverbindung (2) keinen Unterschied beim Umsatz am Gelpunkt und sogar eine deutliche Erniedrigung des finalen Umsatzes verursachte. Trotz des erhöhten Umsatzes, zeichnet sich Verbindung 1 durch eine signifikant schwächere polymerisationsinduzierte Schrumpfspannung aus, während die geringe Schrumpfspannung bei V7 aus dem signifikant geringeren Umsatz hervorgeht. Somit zeigt sich auch hier die Vorteilhaftigkeit von Übertragungsreagenz (1) gegenüber dem literaturbekannten Übertragungsreagenz (2). Referenzverbindung (3) erhöht in V8 zwar die Zeit bis zum Gelpunkt, führt jedoch ebenso wie Verbindung (2) zu einer deutlichen Verringerung des Umsatzes am Gelpunkt und auch zu einem niedrigeren finalen Umsatz (65%). Die auftretende Schrumpfkraft ist zwar geringer als bei V6, dies ist jedoch wie in V7 auf den niedrigeren Umsatz zurückzuführen und zeigt wieder die geringe Wirkung als Übertragungsreagenz.

**Beispiel 8**

*Dynamische-Mechanische Thermoanalyse (DMTA) der hergestellten Photopolymere*

Zur Untersuchung des Glasübergangs wurden analog zu dem obigen Versuch B3 und den Vergleichsversuchen V6, V7 und V8 hergestellte Formulierungen in Silikonformen gegossen und in einem Lichtofen (Modell Lumamat 100, Ivoclar AG) mit Programm 2 (10 min Bestrahlung mit einer Intensität von ca. 20 $mW/cm^2$) polymerisiert. Die Stäbe wurden gewendet und abermals ausgehärtet. Die Probestäbchen wurden geschliffen und dann auf einem Rheometer MCR301 der Firma Anton Paar mit einem CTD(Convection Temperature Control)-Ofen und einer eingesetzten Festkörpereinspannvorrichtung (SRF12 für rechteckige Querschnitte bis 12 mm) vermessen. Die eingestellte Heizrate betrug 2°C/min. Alle Proben wurden von -100°C auf 200°C aufgeheizt und mit einer konstanten Frequenz von 1 Hz und 0,1 % Auslenkung oszilliert. In nachstehender Tabelle 4 sind die Ergebnisse für die Bestimmung des Speichermoduls bei Raumtemperatur ($G'_{(25°C)}$), der Glasübergangstemperatur ($T_G$) und der Halbwertsbreite der Verlustfaktorkurve beim Glasübergang (HWB) angegeben.

***Tabelle 4: Ergebnisse der DMTA***

| **Beispiel** | **Verbindung** | **$G'_{(25°C)}$ [MPa]** | **$T_G$ [°C]** | **HWB [°C]** |
|---|---|---|---|---|
| V9 | - | 1550 | 156 | 161 |
| B4 | (1)[a] | 840 | 57 | 14 |
| V10 | (2)b | 379 | 60 | 55 |
| V11 | (3)[c] | 1510 | 153 | 168 |
| a = 2-((1-Ethoxy-2-methyl-1-oxopropan-2-yl)oxy)acryl-säureethylester<br>b = 2,2-Dimethyl-4-methylenglutarsäuredimethylester<br>c = Bis(2-hydroxy-3-(methacryloyloxy)propyl)-2,2-dimethyl-4-mehylenepentandioat | | | | |

Die in Tabelle 4 gezeigten Daten belegen, dass mit der erfindungsgemäßen Verbindung (1) als Übertragungsreagenz sowohl ein niedrigerer als auch ein deutlich schmalerer Glasübergangsbereich erreicht wird. Diesen Trend kann auch bei Vergleichsbeispiel V10 mit Referenzverbindung (2) festgestellt werden, jedoch bei weitem nicht so signifikant wie in B4 und bei einem gleichzeitig viel stärkeren Abfall des Speichermoduls bei Raumtemperatur. Auch hier zeigen sich die vorteilhaften Eigenschaften von erfindungsgemäßen Polymermaterialien mit der Verbindung (1) gegenüber dem Stand der Technik. Betrachtet man V11 mit der Referenzverbindung (3) erkennt man, dass es zu keiner erheblichen Veränderung der Parameter gegenüber V9 ohne Übertragungsreagenz kam und somit erneut die fehlende Wirkung als Übertragungsreagenz und die bloße Wirkung als Comonomer sichtbar wird.

**Beispiel 9**

*Messung der Schlagzähigkeit (Dynstat-Impact-Test)*

Die Ermittlung der Zähigkeitseigenschaften erfolgte mittels der DYNSTAT-Anordnung entsprechend der DIN 53435, wobei die Schlagzähigkeit (Schlagarbeit) von ungekerbten Prüfkörpern in der Schlagbiegeanordnung ermittelt wurde. Zu diesem Zweck wurden Probestäbe (15 x 10 x 4 mm) aus zu dem obigen erfindungsgemäßen Beispiel 3 und den Vergleichsbeispielen 6, 7 und 8 analogen Formulierungen hergestellt und Dynstat-Impact Tests unter Verwendung eines 10-kpcm-Hammers (10 J) durchgeführt. In der folgenden Tabelle 5 sind die dabei erzielten Werte aufgelistet.

*Tabelle 5: Ergebnisse der Schlagzähigkeitsmessungen*

| Beispiel | Verbindung | Schlagarbeit [kJ/m$^2$][a] |
|---|---|---|
| V12 | - | 5,9 ± 2,3 |
| B5 | (1)[b] | 12, 9 ± 2,3 |
| V13 | (2)[c] | 6,1 ± 2,1 |
| V14 | (3)[d] | 7,7 ± 2,5 |
| a = Normiert auf Breite und Dicke<br>b = 2-((1-Ethoxy-2-methyl-1-oxopropan-2-yl)oxy)acryl-säureethylester<br>c = 2,2-Dimethyl-4-methylenglutarsäuredimethylester<br>d = Bis(2-hydroxy-3-(methacryloyloxy)propyl)-2,2-dimethyl-4-mehylenepentandioat | | |

Es ist ersichtlich, dass nur mit der erfindungsgemäßen Verbindung (1) in B5 eine deutliche Schlagzähigkeitserhöhung erzielt werden konnte, während die Referenzverbindungen (2) und (3) in V13 und V14 die Schlagzähigkeit zwar erhöhen, der Anstieg jedoch innerhalb der Fehlertoleranz liegt. Dies belegt erneut die vorteilhaften mechanischen Eigenschaften der erfindungsgemäßen Polymermaterialien im Vergleich zum Stand der Technik.

**Beispiel 10**

*Zugversuch mit Photopolymerisaten*

Um weitere mechanische Kenndaten (maximale Zugspannung und Bruchdehnung) zu erhalten, wurden Zugversuche durchgeführt. Dazu wurden Probenstäbe nach ISO 527 aus dem obigen erfindungsgemäßen Beispiel B3 und den Vergleichsbeispielen V6, V7 und V8 analogen Formulierungen hergestellt und an einer Zwick Z050-Apparatur der Firma Zwick vermessen. Die Versuche selbst wurden mit einer Traversengeschwindigkeit von 5 mm/min durchgeführt.

*Tabelle 6: Ergebnisse der Zugversuche*

| Beispiel | Verbindung | Maximale Zugspannung [MPa] | Dehnung am Bruch [%] |
|---|---|---|---|
| V15 | - | 66,4 ± 5,4 | 7,7 ± 0,8 |
| B6 | (1)[a] | 31,0 ± 1,0 | 26,9 ± 2,8 |
| V16 | (2)[b] | 21,8 ± 2,7 | 11,5 ± 2,9 |
| V17 | (3)[c] | 68,7 ± 4,3 | 7,98 ± 1,2 |
| a = 2-((1-Ethoxy-2-methyl-1-oxopropan-2-yl)oxy)acryl-säureethylester<br>b = 2,2-Dimethyl-4-methylenglutarsäuredimethylester<br>c = Bis(2-hydroxy-3-(methacryloyloxy)propyl)-2,2-dimethyl-4-mehylenepentandioat | | | |

Die Ergebnisse in Tabelle 6 zeigen, dass der erfindungsgemäße Einsatz von Verbindung (1) zwar zu einer Verringerung der maximalen Zugspannung führt, jedoch die Dehnung am Bruch um 350% gesteigert wird. Referenzverbindung (2) senkt die maximale Zugspannung noch signifikanter und steigert gleichzeitig die Dehnung am Bruch nur marginal. Auch in dieser Hinsicht ermöglicht Verbindung (1) deutlich vorteilhafte mechanische Eigenschaften gegenüber Vergleichsverbindung (2). Referenzverbindung (3) zeigt wie bisher weder signifikante Änderungen bei der maximalen Zugspannung noch bei der Dehnung am Bruch.

**Patentansprüche**

1. Radikalisch polymerisierbare Zusammensetzung zur Anwendung bei der intraoralen Restauration geschädigter Zähne, die mindestens einen Vinylether gemäß der allgemeinen Formel II

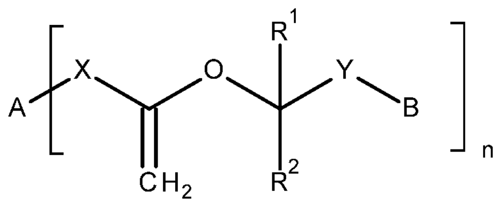

Formel II

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$, substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, $-CON(R^3)$- oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, $-CON(R^3)$- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3,

oder
einen Vinylether der Formel III,

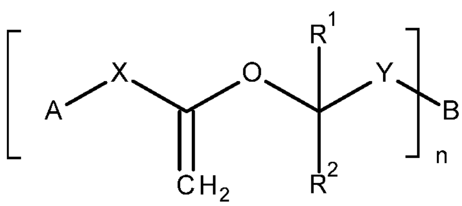

Formel III

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$ substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, $-CON(R^3)$- oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen

enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, -CON($R^3$)- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3, und

mindestens ein (Meth)acrylat mit zwei oder mehr radikalisch polymerisierbaren Gruppen oder eine Mischung von (Meth)acrylaten mit einer und (Meth)acrylaten mit zwei oder mehr radikalisch polymerisierbaren Gruppen enthält.

**2.** Verwendung einer radikalisch polymerisierbare Zusammensetzung, die mindestens einen Vinylether gemäß der allgemeinen Formel II

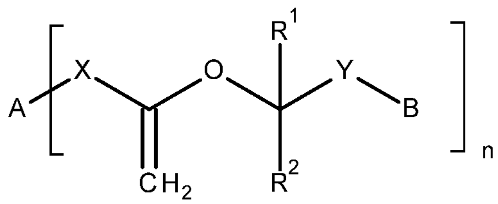

Formel II

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$, substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, -CON($R^3$)- oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, -CON($R^3$)- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3,

oder
einen Vinylether der Formel III,

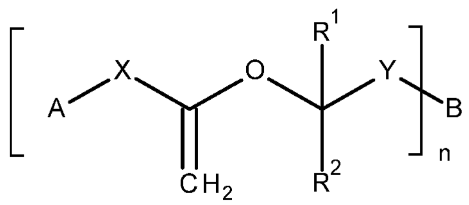

Formel III

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$ substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, $-CON(R^3)$- oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, $-CON(R^3)$- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3, und

mindestens ein (Meth)acrylat mit zwei oder mehr radikalisch polymerisierbaren Gruppen oder eine Mischung von (Meth)acrylaten mit einer und (Meth)acrylaten mit zwei oder mehr radikalisch polymerisierbaren Gruppen enthält, als Material zur extraoralen Herstellung oder Reparatur von Dentalrestaurationen oder als medizinischer Werkstoff zur Herstellung von Medizinprodukten für die Chirurgie oder für die Augenheilkunde.

**3.** Zusammensetzung zur Anwendung gemäß Anspruch 1 oder Verwendung nach Anspruch 2, wobei die Zusammensetzung zusätzlich mindestens einen Initiator für die radikalische Polymerisation enthält.

**4.** Zusammensetzung zur Anwendung gemäß Anspruch 1 oder 3 oder Verwendung nach Anspruch 2 oder 3, wobei die Zusammensetzung als (Meth)acrylat mit einer radikalisch polymerisierbaren Gruppe Methyl-, Butyl-, Tetrahydrofurfuryl- oder Isobornyl(meth)acrylat, p-Cumyl-phenoxyethylenglycolmethacrylat (CMP-1E) oder eine Mischung davon enthält.

**5.** Zusammensetzung zur Anwendung gemäß Anspruch 1, 3 oder 4 oder Verwendung nach einem der Ansprüche 2 bis 4, wobei die Zusammensetzung als (Meth)acrylat mit zwei oder mehr radikalisch polymerisierbaren Gruppen Bisphenol-A-di(meth)-acrylat, 2-[4-(2-(Meth)acryloyloxyethoxyethoxy)phenyl]-2-[4-(2-methacryloyloxyethoxy)phenyl]-propan), 1,6-Bis-[2-(meth)acryloyloxyethoxycarbonylamino]-2,2,4-trimethylhexan (UD(M)A), Tri- oder Tetraethylenglycoldi(meth)acrylat, Glycerintri(meth)acrylat, 1,10-Decandioldi(meth)acrylat ($D_3MA$), 1,12-Dodecandioldi(meth)acrylat oder eine Mischung davon enthält.

**6.** Zusammensetzung zur Anwendung gemäß einem der Ansprüche 1 oder 3 bis 5 oder Verwendung nach einem der Ansprüche 2 bis 5, wobei die Zusammensetzung zusätzlich mindestens einen anorganischen Füllstoff enthält.

**7.** Zusammensetzung zur Anwendung gemäß einem der Ansprüche 1 oder 3 bis 6 oder Verwendung nach einem der Ansprüche 2 bis 6, wobei die Zusammensetzung

a) 0,1 bis 30 Gew.-%, bevorzugt 1 bis 25 Gew.-% und besonders bevorzugt 1 bis 15 Gew.-% mindestens einer Verbindung gemäß der allgemeinen Formel II oder III,
b) 0,01 bis 5 Gew.-%, besonders bevorzugt 0,1 bis 3,0 Gew.-% mindestens eines Initiators für die radikalische Polymerisation,
c) 1 bis 90 Gew.-%, bevorzugt 1 bis 80 Gew.-% und besonders bevorzugt 10 bis 80 Gew.-% mindestens eines (Meth)acrylates mit zwei oder mehr radikalisch polymerisierbaren Gruppen oder einer Mischung von (Meth)acrylaten mit einer und (Meth)acrylaten mit zwei oder mehr radikalisch polymerisierbaren Gruppen,
d) 0 bis 85 Gew.-%, bevorzugt 0 bis 80 Gew.-% und besonders bevorzugt 0 bis 75 Gew.-% mindestens eines Füllstoffs enthält, jeweils bezogen auf die Gesamtmasse der Zusammensetzung.

**8.** Verwendung nach einem der Ansprüche 2 bis 7 zur Herstellung von künstlichen Zähnen, Prothesen, Inlays, Onlays, Kronen oder Brücken.

**9.** Verwendung nach einem der Ansprüche 2 bis 8 zur Herstellung von Formkörpern durch Gießen, Pressen oder 3D-Druck.

**10.** Verwendung nach einem der Ansprüche 2 bis 7 als Werkstoff zur Herstellung von Implantaten für Gehörprothesen, Knorpel- oder Knochenersatzteile oder zur Herstellung von Intraokularlinsen.

**11.** Verwendung einer Verbindung der Formel II

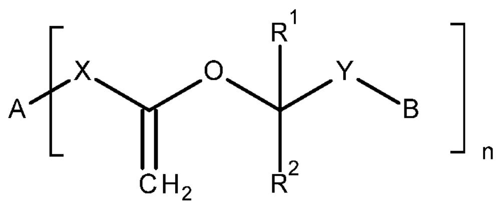

Formel II

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$, substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, $-CON(R^3)$- oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, $-CON(R^3)$- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3,

oder
einer Verbindung der Formel III,

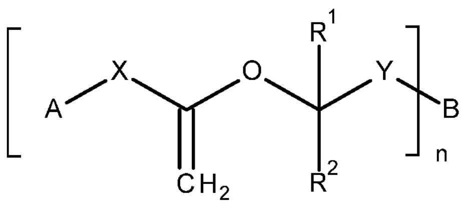

Formel III

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$ substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, $-CON(R^3)-$ oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, $-CON(R^3)-$ oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3,

zur Verringerung der Polymerisationsschrumpfungsspannung von Polymerisaten.

**12.** Verwendung einer Verbindung der Formel II

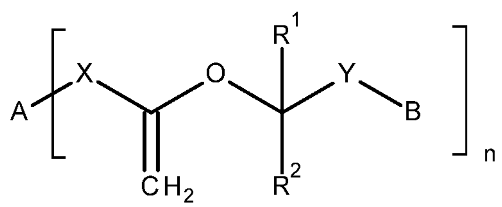

Formel II

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$, substituiert sein kann, der durch 1 bis 2 Urethangruppen, Estergruppen, O und/oder S unterbrochen sein kann und der 1 bis 2 1,4Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, $-CON(R^3)-$ oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis

2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, -CON($R^3$)- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3,

oder
einer Verbindung der Formel III,

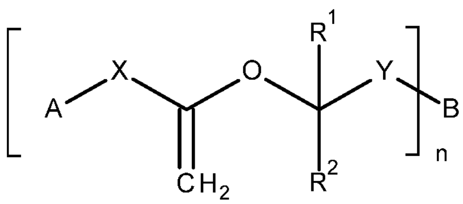

Formel III

in der die Variablen die folgenden Bedeutungen haben:

A ein cycloaliphatischer, linearer oder verzweigter aliphatischer $C_1$-$C_{12}$-Kohlenwasserstoffrest, der durch 1 bis 3 Substituenten ausgewählt aus $-CH_3$, $-C_2H_5$, -OH und/oder $-OCH_3$ substituiert sein kann, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
X -COO-, -CON($R^3$)- oder entfällt, wobei die Bindung an A über O oder N erfolgt und wobei X entfällt, wenn A ein aromatischer Kohlenwasserstoffrest ist;
B ein cycloaliphatischer, linearer oder verzweigter aliphatischer $C_1$-$C_{12}$-Kohlenwasserstoffrest, der durch 1 bis 2 Urethangruppen, Estergruppen, O und/oder S unterbrochen sein kann und der 1 bis 2 1,4-Phenylengruppen enthalten kann, ein aromatischer $C_6$-$C_{14}$-Kohlenwasserstoffrest, der durch 1 bis 3 Substituenten ausgewählt aus $-CH_3$, $-C_2H_5$, -OH, $-OCH_3$ und/oder $-O-COCH_3$ substituiert sein kann, oder eine Kombination davon, wobei die Kohlenwasserstoffreste endständig eine (Meth)acrylatgruppe tragen können;
Y -COO-, -CON($R^3$)- oder entfällt, wobei die Bindung an B über O oder N erfolgt und wobei Y entfällt, wenn B ein aromatischer Kohlenwasserstoffrest ist;
$R^{1-3}$ jeweils unabhängig voneinander Wasserstoff ein cycloaliphatischer, linearer oder verzweigter aliphatischer $C_1$-$C_6$-Kohlenwasserstoffrest, der durch 1 bis 2 Sauerstoffatome unterbrochen sein kann und der durch 1 bis 2 OH-Gruppen substituiert sein kann, oder ein aromatischer $C_6$-$C_{10}$-Kohlenwasserstoffrest, der durch 1 bis 2 OH-Gruppen substituiert sein kann;
n eine ganze Zahl von 1 bis 3,

zur Verbesserung der Schlagzähigkeit von radikalischen Polymerisaten.

## Claims

**1.** Radically polymerizable composition for use in the intraoral restoration of damaged teeth, comprising at least one vinyl ether according to general formula II,

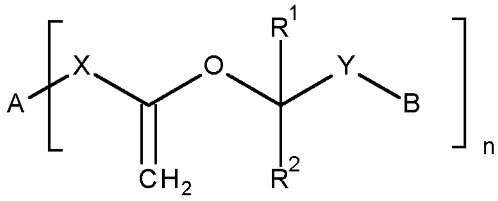

Formula II

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, $-CON(R^3)-$ or is absent, wherein the bonding to A is effected via O or N and wherein X is absent when A is an aromatic hydrocarbon residue;
B cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
Y -COO-, $-CON(R^3)-$ or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3,

or
a vinyl ether of formula III,

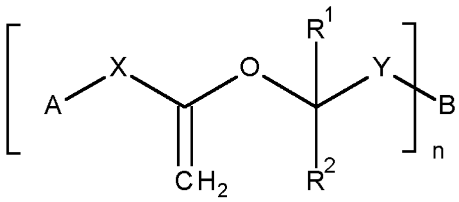

Formula III

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, $-CON(R^3)-$ or is absent, wherein the bonding to A is effected via O or N and wherein X is absent when A is an aromatic hydrocarbon residue;
B a cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;

Y -COO-, -CON($R^3$)- or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3, and

at least one (meth) acrylate with two or more radically polymerizable groups or a mixture of (meth)acrylates with one and (meth)acrylates with two or more radically polymerizable groups.

**2.** Use of a radically polymerizable composition comprising at least one vinyl ether according to general formula II,

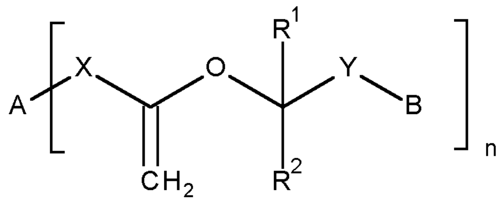

Formula II

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, -CON($R^3$)- or is absent, wherein the bonding to A is effected via O or N and wherein X is absent when A is an aromatic hydrocarbon residue;
B cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
Y -COO-, -CON($R^3$)- or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3,

or
a vinyl ether of formula III,

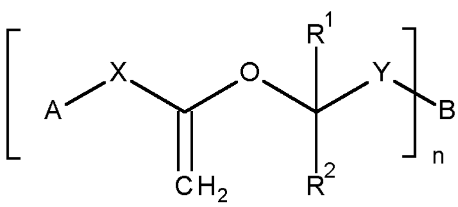

Formula III

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or -O-$COCH_3$, or a combination thereof, wherein the hydrocarbon residues can terminally bear a (meth)acrylate group;
X -COO-, -CON($R^3$)- or is absent, wherein the bonding to A is effected via O or N and wherein X is absent when A is an aromatic hydrocarbon residue;
B a cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or -O-$COCH_3$, or a combination thereof, wherein the hydrocarbon residues can terminally bear a (meth)acrylate group;
Y -COO-, -CON($R^3$)- or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3, and

at least one (meth) acrylate with two or more radically polymerizable groups or a mixture of (meth)acrylates with one and (meth)acrylates with two or more radically polymerizable groups, as a material for the extraoral production or repair of dental restorations or as a medical material for the production of medical products for surgery or for ophthalmology.

3. Composition for use according to claim 1 or use according to claim 2, wherein the composition additionally comprises at least one initiator for the radical polymerization.

4. Composition for use according to claim 1 or 3 or use according to claim 2 or 3, wherein the composition comprises as (meth)acrylate with one radically polymerizable group methyl, butyl, tetrahydrofurfuryl or isobornyl (meth)acrylate, p-cumylphenoxyethylene glycol methacrylate (CMP-1E) or a mixture thereof.

5. Composition for use according to claim 1, 3 or 4 or use according to one of claims 2 to 4, wherein the composition comprises as (meth)acrylate with two or more radically polymerizable groups bisphenol A di(meth)acrylate, 2-[4-(2-(meth)acryloyloxyethoxyethoxy)phenyl]-2-[4-(2-methacryloyloxyethoxy)phenyl]-propane), 1,6-bis-[2-(meth)acryloyloxyethoxycarbonylamino]-2,2,4-trimethylhexane (UD(M)A), trior tetraethylene glycol di(meth)acrylate, glycerol tri(meth)acrylate, 1,10-decanediol di(meth)acrylate ($D_3MA$), 1,12-dodecanediol di(meth)acrylate or a mixture thereof.

6. Composition for use according to one of claims 1 or 3 to 5 or use according to one of claims 2 to 5, wherein the composition additionally comprises at least one inorganic filler.

7. Composition for use according to one of claims 1 or 3 to 6 or use according to one of claims 2 to 6, wherein the composition comprises

a) 0.1 to 30 wt.-%, preferably 1 to 25 wt.-% and particularly preferably 1 to 15 wt.-% of at least one compound according to general formula II or III,
b) 0.01 to 5 wt.-%, particularly preferably 0.1 to 3.0 wt.-% of at least one initiator for the radical polymerization,
c) 1 to 90 wt.-%, preferably 1 to 80 wt.-% and particularly preferably 10 to 80 wt.-% of at least one (meth)acrylate with two or more radically polymerizable groups or a mixture of (meth)acrylates with one and (meth)acrylates with two or more radically polymerizable groups,
d) 0 to 85 wt.-%, preferably 0 to 80 wt.-% and particularly preferably 0 to 75 wt.-% of at least one filler, in each case relative to the total mass of the composition.

8. Use according to one of claims 2 to 7 for the production of artificial teeth, prostheses, inlays, onlays, crowns or bridges.

9. Use according to one of claims 2 to 8 for the production of shaped bodies by casting, compression molding or 3D printing.

**10.** Use according to one of claims 2 to 7 as a material for the production of implants for hearing prostheses, cartilage or bone replacements or for the production of intraocular lenses.

**11.** Use of a compound of according to formula II,

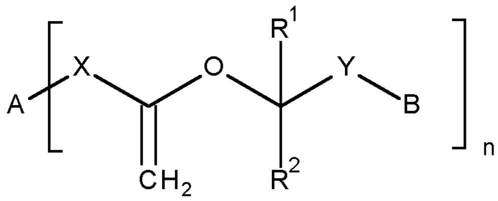

Formula II

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, $-CON(R^3)-$ or is absent, wherein the bonding to A is effected via O or N and wherein X is absent when A is an aromatic hydrocarbon residue;
B cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
Y -COO-, $-CON(R^3)-$ or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3,

or
a vinyl ether of formula III,

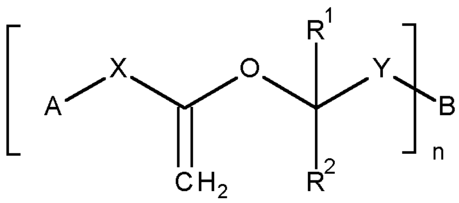

Formula III

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, $-CON(R^3)-$ or is absent, wherein the bonding to A is effected via O or N and wherein X is absent when A is an aromatic hydrocarbon residue;

B a cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
Y -COO-, $-CON(R^3)-$ or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3,

to reduce the polymerization shrinkage stress of polymers.

**12.** Use of a compound of according to formula II,

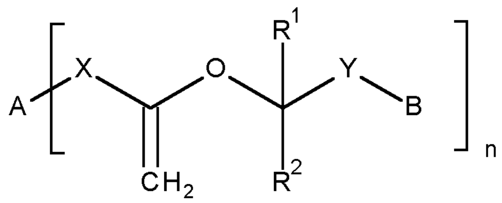

Formula II

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, $-CON(R^3)-$ or is absent, wherein the bonding to A is effected via O or N and wherein X is absent when A is an aromatic hydrocarbon residue;
B cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
Y -COO-, $-CON(R^3)-$ or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3,

or
a vinyl ether of formula III,

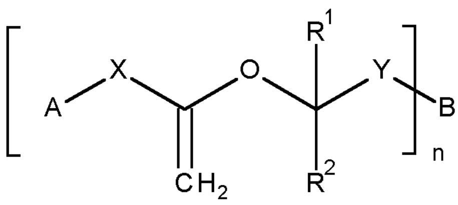

Formula III

in which the variables have the following meanings:

A a cycloaliphatic, linear or branched aliphatic $C_1$-$C_{12}$ hydrocarbon residue, which can be substituted by 1 to 3 substituents selected from $-CH_3$, $-C_2H_5$, -OH and/or $-OCH_3$, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
X -COO-, $-CON(R^3)-$ or is absent, wherein the bonding to A is effected via O or N and wherein X is absent when A is an aromatic hydrocarbon residue;
B a cycloaliphatic, linear or branched aliphatic $C_1$-$C_{12}$ hydrocarbon residue, which can be interrupted by 1 to 2 urethane groups, ester groups, O and/or S and which can comprise 1 to 2 1,4-phenylene groups, an aromatic $C_6$-$C_{14}$ hydrocarbon residue, which can be substituted by 1 to 3 substituents selected from $-CH_3$, $-C_2H_5$, -OH, $-OCH_3$ and/or $-O-COCH_3$, or a combination thereof, wherein the hydrocarbon residues can terminally bear a (meth)acrylate group;
Y -COO-, $-CON(R^3)-$ or is absent, wherein the bonding to B is effected via O or N and wherein Y is absent when B is an aromatic hydrocarbon residue;
$R^{1-3}$ in each case independently of one another hydrogen, a cycloaliphatic, linear or branched aliphatic $C_1$-$C_6$ hydrocarbon residue, which can be interrupted by 1 to 2 oxygen atoms and which can be substituted by 1 to 2 OH groups, or an aromatic $C_6$-$C_{10}$ hydrocarbon residue, which can be substituted by 1 to 2 OH groups;
n an integer from 1 to 3,

to improve the impact resistance of radical polymers.

## Revendications

**1.** Composition polymérisable par voie radicalaire pour utilisation dans la restauration intraorale de dents abimées, qui contient au moins un éther vinylique de formule générale II :

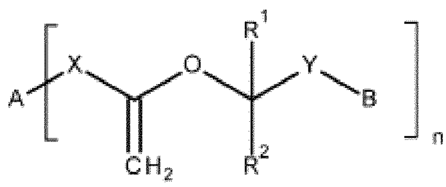

Formule II

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)-$ ou ne représente rien, étant entendu que la liaison vers le reste A s'effectue par l'intermédiaire de l'atome d'oxygène ou d'azote et que X ne représente rien quand le reste A est un reste

d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou -CON($R^3$)- ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;

et n est un nombre entier valant de 1 à 3 ;
ou un éther vinylique de formule III :

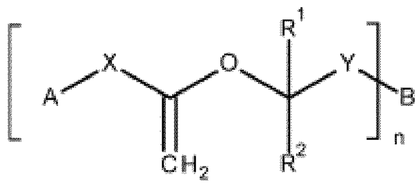

Formule III

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou -CON($R^3$)- ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou -CON($R^3$)- ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;
et n est un nombre entier valant de 1 à 3 ;
et au moins un (méth)acrylate comportant deux groupes polymérisables par voie radicalaire ou plus, ou un mélange de (méth)acrylates comportant un groupe polymérisable par voie radicalaire et de (méth)acrylates en comportant deux ou plus.

**2.** Utilisation d'une composition polymérisable par voie radicalaire qui contient au moins un éther vinylique de formule générale II :

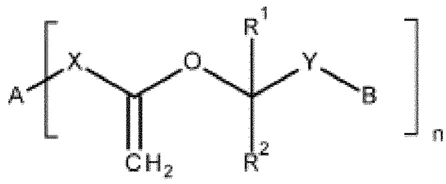

Formule II

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)$- ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou $-CON(R^3)$- ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;

et n est un nombre entier valant de 1 à 3 ;
ou un éther vinylique de formule III :

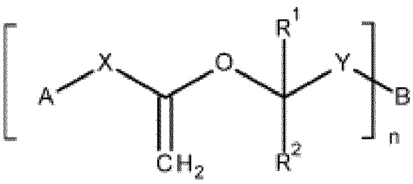

Formule III

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)$- ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut

être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi -$CH_3$, -$C_2H_5$, -OH, -$OCH_3$ et/ou -O-$COCH_3$, ou une combinaison de tels restes, étant entendu que ces restes d'hydrocarbure peuvent porter un groupe acrylate ou méthacrylate en position terminale ;

Y représente -COO- ou -CON($R^3$)- ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;

$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;

et n est un nombre entier valant de 1 à 3 ;

et au moins un (méth)acrylate comportant deux groupes polymérisables par voie radicalaire ou plus, ou un mélange de (méth)acrylates comportant un groupe polymérisable par voie radicalaire et de (méth)acrylates en comportant deux ou plus,

en tant que matériau pour la fabrication ou la réparation extraorale de restaurations dentaires ou en tant que matériau médicinal pour la fabrication de produits médicaux pour chirurgie ou ophtalmologie.

**3.** Composition pour utilisation conforme à la revendication 1, ou utilisation conforme à la revendication 2, étant entendu que la composition contient en outre au moins un amorceur de polymérisation par voie radicalaire.

**4.** Composition pour utilisation conforme à la revendication 1 ou 3, ou utilisation conforme à la revendication 2 ou 3, étant entendu que la composition contient, en tant que (méth)acrylate(s) comportant un groupe polymérisable par voie radicalaire, du (méth)acrylate de méthyle, de butyle, de tétrahydrofurfuryle ou d'isobornyle, du méthacrylate de para-cumyl-phénoxy-éthylèneglycol (CMP-1E), ou un mélange de ceux-ci.

**5.** Composition pour utilisation conforme à la revendication 1, 3 ou 4, ou utilisation conforme à l'une des revendications 2 à 4, laquelle composition contient, en tant qu'acrylate ou méthacrylate doté de deux groupes polymérisables par voie radicalaire ou plus, du di(méth)acrylate de bisphénol A, du 2-[4-(2-(méth)acryloyloxyéthoxyéthoxy)-phényl]-2-[4-(2-méthacryloyloxy-éthoxy)-phényl]-propane, du 1,6-bis-[2-(méth)acryloyloxy-éthoxycarbonylamino]-2,2,4-triméthylhexane (UD(M)A), du di(méth)acrylate de triéthylèneglycol ou de tétraéthylèneglycol, du tri(méth)acrylate de glycérol, du di(méth)acrylate de décane-1,10-diol ($D_3$MA), du di(méth)acrylate de dodécane-1,12-diol, ou un mélange de ces composés.

**6.** Composition pour utilisation conforme à l'une des revendications 1 et 3 à 5, ou utilisation conforme à l'une des revendications 2 à 5, laquelle composition contient en outre au moins une charge inorganique.

**7.** Composition pour utilisation conforme à l'une des revendications 1 et 3 à 6, ou utilisation conforme à l'une des revendications 2 à 6, laquelle composition contient

a) de 0,1 à 30 % en poids, de préférence de 1 à 25 % en poids, et tout particulièrement de 1 à 15 % en poids d'au moins un composé de formule générale II ou III,

b) de 0,01 à 5 % en poids, en particulier de 0,1 à 3,0 % en poids d'au moins un amorceur de polymérisation par voie radicalaire,

c) de 1 à 90 % en poids, de préférence de 1 à 80 % en poids, et tout particulièrement de 10 à 80 % en poids d'au moins un (méth)acrylate comportant deux groupes polymérisables par voie radicalaire ou plus, ou d'un mélange de (méth)acrylates comportant un groupe polymérisable par voie radicalaire et de (méth)acrylates qui en comportent deux ou plus,

d) et de 0 à 85 % en poids, de préférence de 0 à 80 % en poids, et tout particulièrement de 0 à 75 % en poids d'au moins une charge,

par rapport, dans chaque cas, à la masse totale de la composition.

**8.** Utilisation, conforme à l'une des revendications 2 à 7, pour la fabrication de dents artificielles, de prothèses, d'inlays, d'onlays, de couronnes ou de bridges.

**9.** Utilisation, conforme à l'une des revendications 2 à 8, pour la fabrication de corps façonnés par coulée, compression

ou impression en 3D.

**10.** Utilisation, conforme à l'une des revendications 2 à 7, en tant que matériau pour la fabrication d'implants pour prothèses auditives ou pièces de remplacement de cartilage ou d'os, ou pour la fabrication de lentilles intraoculaires.

**11.** Utilisation d'un composé de formule II :

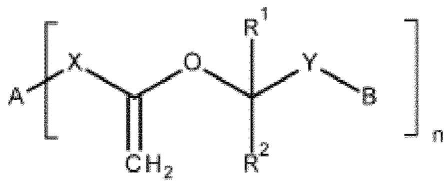

Formule II

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)-$ ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou $-CON(R^3)-$ ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;

et n est un nombre entier valant de 1 à 3 ;
ou d'un composé de formule III :

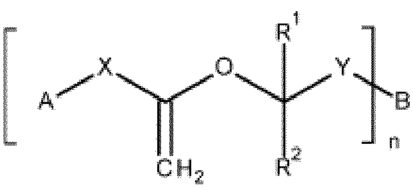

Formule III

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2

groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)-$ ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou $-CON(R^3)-$ ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;
et n est un nombre entier valant de 1 à 3 ;
pour réduire la tension de retrait de polymérisation de produits de polymérisation.

**12.** Utilisation d'un composé de formule II :

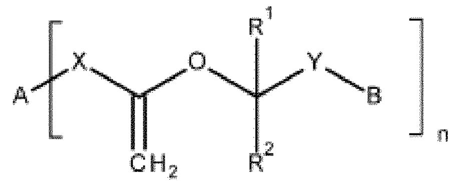

Formule II

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)-$ ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou $-CON(R^3)-$ ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;

et n est un nombre entier valant de 1 à 3 ;
ou d'un composé de formule III :

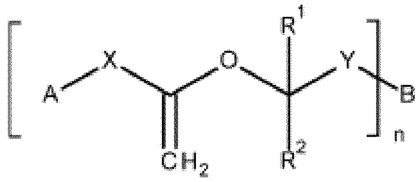

Formule III

dans laquelle les symboles ont les significations suivantes :

A représente un reste d'hydrocarbure cycloaliphatique ou aliphatique linéaire ou ramifié en $C_1$-$C_{12}$, qui peut porter 1 à 3 substituant(s) choisis parmi $-CH_3$, $-C_2H_5$, -OH et/ou $-OCH_3$, qui peut être interrompu par 1 à 2 groupe(s) uréthane, groupe(s) ester, atome(s) d'oxygène et/ou atome(s) de soufre et qui peut comporter 1 à 2 groupe(s) 1,4-phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
X représente -COO- ou $-CON(R^3)-$ ou ne représente rien, étant entendu que la liaison vers le reste A s'effectue par l'intermédiaire de l'atome d'oxygène ou d'azote et que X ne représente rien quand le reste A est un reste d'hydrocarbure aromatique ;
B représente un reste d'hydrocarbure cycloaliphatique ou aliphatique linéaire ou ramifié en $C_1$-$C_{12}$, qui peut être interrompu par 1 à 2 groupe(s) uréthane, groupe(s) ester, atome(s) d'oxygène et/ou atome(s) de soufre et qui peut comporter 1 à 2 groupe(s) 1,4phénylène, un reste d'hydrocarbure aromatique en $C_6$-$C_{14}$, qui peut porter 1 à 3 substituant(s) choisis parmi $-CH_3$, $-C_2H_5$, -OH, $-OCH_3$ et/ou $-O-COCH_3$, ou une combinaison de tels restes, étant entendu que ces restes d'hydrocarbure peuvent porter un groupe acrylate ou méthacrylate en position terminale ;
Y représente -COO- ou $-CON(R^3)-$ ou ne représente rien, étant entendu que la liaison vers le reste B s'effectue par l'intermédiaire de l'atome d'oxygène ou d'azote et que Y ne représente rien quand le reste B est un reste d'hydrocarbure aromatique ;
$R^{1-3}$ représentent chacun, indépendamment l'un de l'autre, un atome d'hydrogène, un reste d'hydrocarbure cycloaliphatique ou aliphatique linéaire ou ramifié en $C_1$-$C_6$, qui peut être interrompu par 1 à 2 atome(s) d'oxygène et qui peut porter 1 à 2 groupe(s) -OH en tant que substituant(s), ou un reste d'hydrocarbure aromatique en $C_6$-$C_{10}$, qui peut porter 1 à 2 groupe(s) -OH en tant que substituant(s) ;
et n est un nombre entier valant de 1 à 3 ;
pour améliorer la résistance au choc de produits de polymérisation par voie radicalaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2694699 A
- US 5932675 A
- EP 3090722 A1
- US 9320685 B2
- WO 8804304 A1
- EP 2965741 A1
- WO 2012112350 A2


### In der Beschreibung aufgeführte Nicht-Patentliteratur


- **H. G. ELIAS.** Makromoleküle. Wiley-VCH, vol. 1, 299-352
- **MOAD et al.** *Polymer,* 2008, vol. 49, 1079-1131
- **P. K. SHAH ; J. W. STANSBURY ; C. N. BOWMAN.** *Polym. Chem.,* 2017
- **MEIJS et al.** *Makromol Chem.,* 1990, vol. 191, 1545-1553
- **G. DEGUEST ; L. BISCHOFF ; C. FRUIT ; F. MARSAIS.** *Org. Lett,* 2007, vol. 9, 1165-1167
- **J. REHBEIN ; S. LEICK ; M. HIERSEMANN.** *Journal of Organic Chemistry,* 2009, vol. 74 (4), 1531-1540
- **D.C. WATTS et al.** *Dental Materials,* 2003, vol. 19, 1-11
- **MOAD, C. L ; MOAD, G ; RIZZARDO, E ; THANG, S. H.** Chain Transfer Activity of ω-Unsaturated Methyl Methacrylate Oligomers. *Macromolecules,* 1996, vol. 29 (24), 7717-7726
- *CHEMICAL ABSTRACTS,* 72869-86-4